# EUROPEAN PATENT SPECIFICATION

(11) **EP 1 137 401 B1**
(45) Date of publication and mention of the grant of the patent: **23.11.2005**
(21) Application number: 99964208.5
(22) Date of filing: 09.12.1999
(51) Int. Cl.: A61K 9/00, A61K 38/18

(54) **ADMINISTRATION OF NEUROTROPHIC AGENTS TO THE CENTRAL NERVOUS SYSTEM**
VERABREICHUNG VON NEUROTROPISCHEN WIRKSTOFFEN IN DAS ZENTRALE NERVENSYSTEM
APPORT D'AGENTS NEUROTROPHIQUES AU SYSTEME NERVEUX CENTRAL

(30) Priority: 09.12.1998 US 208538
(43) Date of publication of application: 04.10.2001
(73) Proprietor: CHIRON CORPORATION, Emeryville, California 94608 (US)
(72) Inventor: Frey, William H., II, White Bear Lake, MN 55127 (US); CHEN, Xuequing, St. Paul - MN 55108 (US); THORNE, Robert Gary, Mineapolis - MN 55408 (US)
(74) Representative: Duckworth, Timothy John
(86) International application number: PCT/US1999/029334
(87) International publication number: WO 2000/033813

(56) References cited:
- EP-A- 0 724 885
- WO-A-91/01739
- WO-A-91/07947
- WO-A-97/31626

## Description

### Field of the Invention

The present invention is directed to use in the manufacture of a medicament for delivering neurotrophic agents to the central nervous system by way of the nasal cavity. Such a medicament can be useful in the treatment of central nervous system and/or brain disorders.

### Background of the Invention

The central nervous system (CNS) includes several tissues and organs, such as the brain, the brain stem, and the spinal cord. Each of these organs and tissues is made up of a variety of different types of cells and subcellular structures, e.g. neurons, glial cells, dendrites, axons, myelin, and various membranes. The CNS is isolated from the external world by several membranes that both cushion and protect these organs, tissues, cells, and structures. For example the membranes that form the blood-brain barrier protect the brain from certain contents of the blood. The blood-cerebrospinal fluid barrier protects other portions of the CNS from many chemicals and microbes.

Access to the CNS for some substances is provided by specialized active transport systems or through passive diffusion through the protective membrane into the CNS. Present methods for delivering desired therapeutic agents to the CNS are typically invasive. For example, a pump implanted in the skull (an intracerebroventricular pump) can effectively deliver a variety of useful compounds to the brain. However, implanting such a pump requires brain surgery, which can entail a variety of serious complications. Certain compounds (e.g. epidural painkillers) can be injected directly through the protective membrane into the CNS. Such injections is, however, impractical for most medications. Better methods for administering desired agents to the CNS, brain, and/or spinal cord are needed.

WO 91/07947 discloses a method for transporting therapeutic neurologic and/or diagnostic neurologic agents to the brain by means of the olfactory neural pathway and a pharmaceutical composition useful in the treatment and diagnosis of brain disorders.

### Summary of the Invention

The present invention relates to use of a neurotrophic agent or biologically active variant thereof in the manufacture of a medicament for delivering a therapeutically effective amount of said neurotrophic agent or said variant thereof to the central nervous system of a mammal by way of a nasal cavity, said medicament comprising a unit dose of 0.1 nmol to 1000 nmol of said neurotrophic agent or said variant thereof, wherein administration of said medicament to said nasal cavity of said mammal provides for transport of said neurotrophic agent or said variant thereof to the central nervous system of said mammal in an amount effective to provide a protective or therapeutic effect on a cell of the central nervous system, said neurotrophic agent being selected from the group consisting of an insulin-like growth factor (IGF), a nerve growth factor (NGF), and a fibroblast growth factor (FGF), and said biologically active variant thereof having at least 70% amino acid sequence identity with the amino acid sequence for said neurotrophic agent and wherein said biologically active variant of IGF is a biologically active variant of insulin-like growth factor I (IGF-I).

The present invention may be used in the administration of a therapeutically effective dose of the neurotrophic agent to the subject's nasal cavity, preferably to the upper third of the nasal cavity. The neurotrophic agent can then be absorbed through a mucosa or epithelium and transported to the central nervous system of the mammal, preferably without crossing the blood brain barrier.

In another embodiment, the therapeutically effective dose of the neurotrophic agent may be administered in a manner such that neurotrophic agent is absorbed through the tissue and transported into the central nervous system of the mammal by a neural pathway and in an amount effective to provide a protective or therapeutic effect on a cell of the central nervous system.

The invention can be used to provide for administration by these routes using a composition that includes a carrier that facilitates absorption of the neurotrophic agent, transport of the neurotrophic agent by a neural pathway, and/or transport of the neurotrophic agent to the CNS, brain, and/or spinal cord. Preferred compositions include one or more of a solubility enhancing additive, a hydrophilic additive, an absorption promoting additive, a cationic surfactant, a viscosity enhancing additive, or a sustained release matrix or composition, a lipid based carrier, preferably a micellar or liposomal composition, a bilayer destablizing additive, or a fusogenic additive.

Neurotrophic agents which may be used in the invention are fibroblast growth factor, particularly basic fibroblast growth factor (bFGF), insulin-like growth factor, particularly insulin-like growth factor-I (IGF-I), and nerve growth factor (NGF).

### Brief Description of the Figures

Figure 1 illustrates a linear relationship between NGF concentration in the olfactory bulb and the doses given by nasal administration.
Figure 2 illustrates a linear relationship between NGF concentration in the olfactory bulb dura membrane and the doses given by nasal administration.
Figure 3 illustrates a linear relationship between NGF concentration in the olfactory epithelium and the doses given by nasal administration.
Figure 4 illustrates a linear relationship between NGF concentration in the cervical spinal cord and the doses given by nasal administration.
Figure 5 illustrates a linear relationship between NGF concentration in the deep cervical lymph nodes and the doses given by nasal administration:

### Detailed Description of the Invention

### Routes of Administration

The invention may be used to administer the agent to tissue innervated by the trigeminal and olfactory nerves and inside the nasal cavity and or sinuses. Such nerve systems can provide a direct connection between the outside environment and the brain, thus providing advantageous delivery of a neurotrophic agent to the CNS, brain, and/or spinal cord.

### The Olfactory Nerve

The invention may be used for administration of an agent to tissue innervated by the olfactory nerve and inside the nasal cavity. Preferably, the agent is delivered to the olfactory area in the upper third of the nasal cavity and particularly to the olfactory epithelium.

Fibers of the olfactory nerve are unmyelinated axons of olfactory receptor cells that are located in the superior one-third of the nasal mucosa. The olfactory receptor cells are bipolar neurons with swellings covered by hair-like cilia which project into the nasal cavity. At the other end, axons from these cells collect into aggregates and enter the cranial cavity at the roof of the nose. Surrounded by a thin tube of pia, the olfactory nerves cross the subarachnoid space containing CSF and enter the inferior aspects of the olfactory bulbs.
Once the agent is dispensed into the nasal cavity, the agent can undergo transport through the nasal mucosa and into the olfactory bulb and interconnected areas of the brain, such as the hippocampal formation, amyedaloid nuclei, nucleus basalis of Meynert, locus ceruleus, the brain stem and the like.

### The Trigeminal Nerve

The invention may also be used for administration of an agent to tissue innervated by the trigeminal nerve and inside the nasal cavity. Within the nasal cavity, the trigeminal nerve innervates mainly the inferior two-thirds of the nasal mucosa. The trigeminal nerve has three major branches, the ophthalmic nerve, the maxillary nerve, and the mandibular nerve. The invention can be used to administer the agent to tissue within the nasal cavity innervated by one or more of these branches.

### The Ophthalmic Nerve and its Branches

The invention can be used administer the agent to tissue within the nasal cavity and/or sinuses innervated by the ophthalmic nerve branch of the trigeminal nerve. The ophthalmic nerve has three branches known as the nasociliary nerve, the frontal nerve, and the lacrimal nerve. The anterior ethmoidal nerve, a branch of the nasociliary nerve, innervates, among other tissues, the ethmoidal sinus and regions of the inferior two-thirds of the nasal mucosa, including the anterior portion of the nasal septum and the lateral wall of the nasal cavity. Preferably, the invention can be used to administer the agent to tissue innervated by the anterior ethmoidal nerve.

### The Maxillary Nerve and its Branches

The invention can be used to administer the agent to tissue within the nasal cavity and/or sinuses innervated by the maxillary nerve branch of the trigeminal nerve. The maxillary nerve has several branches that innervate the nasal cavity and sinuses, including the nasopalatine nerve, the greater palatine nerve, the posterior superior alveolar nerves, the middle superior alveolar nerve, and the interior superior alveolar nerve. The maxillary sinus is innervated by the posterior, middle and anterior superior alveolar nerves. The mucous membrane of the nasal septum is supplied chiefly by the nasopalatine nerve, and the lateral wall of the nasal cavity is supplied by the greater palatine nerve. Preferably, the invention can be used to administer the agent to tissue innervated by the nasopalatine nerve and/or greater palatine nerve.

### Neurotrophic Agents for Delivery to the Central Nervous System

A variety of different neurotrophic agents can be administered to the central nervous system using the invention. In general, the invention can be used to administer a neurotrophic agent that can be employed for prevention or treatment of a disease or disorder affecting the CNS, brain, and/or spinal cord; that can promote growth, regeneration or survival of a cell or tissue in the CNS, brain, and/or spinal cord; or the like. As used herein ''neurotrophic agent" refers to proteins such as growth factors, neurotrophins, neurotrophic factors, and the like, that have these activities.

In particular, neurotrophic agent includes nerve growth factor (NGF), neurotrophins 3, 4, and/or 5 (NT-3. NT-4 and/or NT-5), brain-derived neurotrophic factor (BDNF), fibroblast growth factors (FGFs, e.g., basic fibroblast growth factor), insulin, insulin-like growth factors (IGFs, e.g., IGF-I and/or IGF-II), ciliary neurotrophic factor (CNTF), glia-derived neurotrophic factor (GDNF), glia-derived nexin, activity dependent neurotrophic factor and combinations thereof.

Certain neurotrophic agents are not or are only poorly transported across the blood-brain barrier. For such agents, an effective amount of the neurotrophic agent does not readily, and may not ever, cross the blood-brain barrier. The present invention can be used to effectively deliver such neurotrophic agents to the CNS, brain, and/or spinal cord.

Administration of neurotrophic agents using the invention can more effectively deliver the neurotrophic agent to the CNS, brain and/or spinal cord, can decrease the amount of neurotrophic agent administered outside the CNS, brain and/or spinal cord, and, can preferably, decrease the undesirable systemic effects of the neurotrophic agent. More effective or efficient delivery of the neurotrophic agent to the CNS, brain and/or spinal cord can decrease the total dose of neurotrophic agent administered. Alternatively such effective delivery of neurotrophic agent can decrease the amount of neurotrophic agent that reaches undesired destinations within the subject but outside the CNS, brain and/or spinal cord. This more effective delivery results in less of such a neurotrophic agent in locations within the subject where it can have undesirable effects.

### IGF-I

The term "IGF-I" as used herein refers to insulin-like growth factor I (IGF-I), a single chain peptide having 70 amino acids and a molecular weight of about 7,600 daltons. Insulin-like growth factor I stimulates mitosis and growth processes associated with cell development.

In one embodiment of the invention, increasing in the amount of IGF-I to a therapeutically effective level is achieved via administration of a pharmaceutical composition including a therapeutically effective dose. The IGF-I to be administered can be from any animal species including, but not limited to, rodent, avian, canine, bovine, porcine, equine, and, preferably, human. Preferably the IGF-I is from a mammalian species, and more preferably is from a mammal of the same species as the mammal undergoing treatment.

Biologically active variants of IGF-1 are also encompassed by the method of the present invention. Such variants should retain IGF-I activities, particularly the ability to bind to IGF-I receptor sites. IGF-I activity may be measured using standard IGF-I bioassays. Representative assays include known radioreceptor assays using placental membranes (see, e.g., U.S. Patent No. 5,324,639; Hall *et al.* (1974) *J. Clin. Endocrinol. and Metab.* 39:973-976; and Marshall *et al.* (1974) *J. Clin. Endocrinol. and Metab.* 39:283-292), a bioassay that measures the ability of the molecule to enhance incorporation of tritiated thymidine, in a dose-dependent manner, into the DNA of BALB/c 3T3 fibroblasts (see, e.g.. Tamura *et al.* (1989) *J. Biol. Chem.* 262:5616-3621). Preferably, the variant has at least the same activity as the native molecule.

Suitable biologically active variants can be IGF-I fragments, analogues, and derivatives. By "IGF-I fragment", is intended a protein consisting of only a part of the intact IGF-I sequence and structure, and can be a C-terminal deletion or N-terminal deletion of IGF-I. By "analogue" is intended an analogue of either IGF-I or an IGF-I fragment that include a native IGF-I sequence and structure having one or more amino acid substitutions, insertions, or deletions. Peptides having one or more peptoids (peptide mimics) are also encompassed by the term analogue (see e.g. International Publication No. WO 91/04282). By "derivative" is intended any suitable modification of IGF-I, IGF-I fragments, or their respective analogues, such as glycosylation, phosphorylation, or other addition of foreign moieties, so long as the IGF-I activity is retained. Methods for making IGF-I fragments, analogues, and derivatives are available in the art. See generally U.S. Patent Nos. 4,738,921, 5,158,875, and 5,077,276; International Publication Nos. WO 85/00831, WO 92/04363, WO 87/01038, and WO 89/05822: and European Patent Nos. EP 135094, EP 123228, and EP 128733:

IGF-I variants will have at least 70%, preferably 80%, more preferably 85%, even more preferably 90% to 95% or more, and most preferably 98% or more amino acid sequence identity to the amino acid sequence of the reference IGF-I molecule. A variant may, for example, differ by as few as 1 to 10 amino acid residues, such as 6-10. as few as 5, as few as 4, 3, 2, or even 1 amino acid residue.

By "sequence identity" is intended the same amino acid residues are found within the variant sequence and a reference sequence when a specified, contiguous segment of the amino acid sequence of the variant is aligned and compared to the amino acid sequence of the reference sequence. Methods for sequence alignment and for determining identity between sequences are well known in the art. See, for example, Ausubel *et al.,* eds. (1995) *Current Protocols in Molecular Biology,* Chapter 19 (Greene Publishing and Wiley-Interscience, New York); and the ALIGN program (Dayhoff (1978) in *Atlas of Prorein Sequence and Structure* 5:Suppl. 3 (National Biomedical Research Foundation, Washington. D.C.). A number of algorithms are available for aligning sequences and determining sequence identity and include, for example, the homology alignment algorithm of Needleman *et al.* (1970) *J. Mol. Biol.* 48:443; the local homology algorithm of Smith *et al.* (1981) *Adv. Appl. Math.* 2:482; the search for similarity method of Pearson *et al.* (1988) *Proc. Natl. Acad. Sci.* 85:2444; the Smith-Waterman algorithm *(Meth. Mol. Biol.* 70:173-187 (1997): and BLASTP, BLASTN, and BLASTX algorithms (see Altschul *et al.* (1990) *J. Mol. Biol. 215*:403-410). Computerized programs using these algorithms are also available, and include, but are not limited to: GAP, BESTFIT. BLAST, FASTA, and TFASTA, available in the Genetics Computing Group (GCG) package, Version 8, Madison, Wisconsin. USA; and CLUSTAL in the PC/Gene program by Intelligenetics, Mountain View, California. Preferably, the sequence identity is determined using the default parameters determined by the program.

With respect to optimal alignment of two amino acid sequences, the contiguous segment of the variant amino acid sequence may have additional amino acid residues or deleted amino acid residues with respect to the reference amino acid sequence. The contiguous segment used for comparison to the reference amino acid sequence will include at least 20 contiguous amino acid residues, and may be 30, 40, 50, or more amino acid residues. Corrections for increased sequence identity associated with inclusion of gaps in the variant's amino acid sequence can be made by assigning gap penalties.

When considering percentage of amino acid sequence identity, some amino acid residue positions may differ as a result of conservative amino acid substitutions, which do not affect properties of protein function. In these instances, percent sequence identity may be adjusted upwards to account for the similarity in conservatively substituted amino acids. Such adjustments are well known in the art. See, for example, Meyers & Miller (1988) *ComputerApplic. Biol. Sci.* 4:11-17.

The art provides substantial guidance regarding the preparation and use of such IGF-I variants, as discussed further below. A fragment of IGF-I will generally include at least about 10 contiguous amino acid residues of the full-length molecule, preferably about 15-25 contiguous amino acid residues of the full-length molecule, and most preferably about 20-50 or more contiguous amino acid residues of full-length IGF-I.

Several IGF-I analogues and fragments are known in the art and include those described in, for example, *Proc. Nutl. Acad Sci. USA* 83 (1986) 4904-4907; *Biochem. Biophys. Res. Commun.* 149 (1987) 398-404;*J. Biol. Chem.* 263 (1988) 6233-6239; *Biochem. Biophys. Res. Commun.* 165 (1989) 766-771; Forsbert *et al.* (1990) *Biochem. J.* 271:357-363; U.S. Patent Nos. 4,876,242 and 5,077,276; and International Publication Nos. WO 87/01038 and WO 89/05822. Representative analogues include one with a deletion of Glu-3 of the mature molecule, analogues with up to 5 amino acids truncated from the N-terminus, an analogue with a truncation of the first 3 N-terminal amino acids (referred to as des(1-3)-IGF-I, des-IGF-I, tIGF-I, or brain IGF), and an analogue including the first 17 amino acids of the B chain of human insulin in place of the first 16 amino acids of human IGF-I.

The IGF-I used in the present invention can be in its substantially purified, native, recombinantly produced, or chemically synthesized forms. IGF-I can be isolated and purified from serum or plasma (see Phillips (1980) *New Eng. J Med.* 302: 371-380, and European Patent No. EP 123,228). IGF-I can also be chemically synthesized by the solid phase method (see Li *et al.* (1983) *Proc. Natl. Acad. Sci. USA* 80: 2216-2220).

Genetic engineering by recombinant DNA techniques can be the most efficient way of producing IGF-I. The human DNA sequence encoding IGF-I is known and can be introduced into host cells for expression. IGF-I can be produced by recombinant DNA techniques in *E*. *coli.* yeast, insect, and mammalian cells. Secreted IGF-I can be made by adding a signal sequence to the DNA sequence encoding IGF-I. In addition, the DNA sequence encoding IGF-I can be manipulated to make IGF-I fragments, analogues, or derivatives. Such recombinant DNA techniques are generally available in the art. See, for example, International Publication No. WO 96/07424, where recombinant human IGF-I protein is produced in yeast. IGF-I can also be recombinantly produced in the yeast strain *Pichia pastoris* and purified essentially as described in U.S. Patent Nos. 5,324,639, 5,324,660, and 5,650,496 and International Publication No. WO 96/40776.

### FGF

By the term "FGF," as used herein, is meant a fibroblast growth factor protein such as FGF-1, FGF-2, FGF-4, FGF-6, FGF-8, FGF-9 or FGF-98, or a biologically active fragment or mutein thereof. Typically, the FGF is human (h) FGF-1, bovine (b) FGF-1, hFGF-2, bFGF-2, hFGF-4 or hFGF-5. In an alternative embodiment, the active agent in the unit does is hFGF-6, hFGF-8, hFGF-9 or hFGF-98. In one embodiment of the invention, increasing in the amount of FGF to a therapeutically effective level is achieved via administration of a pharmaceutical composition including a therapeutically effective dose. The FGF to be administered can be from any animal species including, but not limited to, rodent, avian, canine, bovine, porcine, equine, and, preferably, human. Preferably the FGF is from a mammalian species, and more preferably is from a mammal of the same species as the mammal undergoing treatment.

The amino acid sequences and method for making many of the FGFs that are employed in a unit dose in pharmaceutical composition used in the present invention are well known in the art. In particular, references disclosing the amino acid sequence and recombinant expression of FGF 1-9 and FGF-98 are discussed sequentially below.

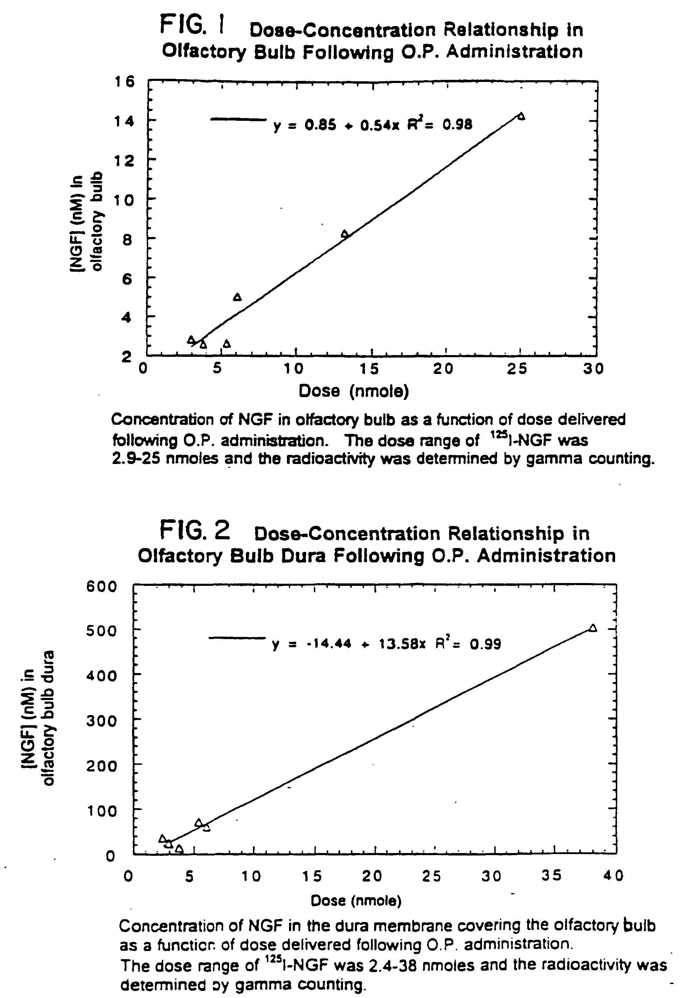

FGF-1 : The amino acid sequence of HFGF-I and a method for its recombinant expression are disclosed in U.S. Patent No. 5,604,293 (Fiddes), entitled "Recombinant Human Basic Fibroblast Growth Factor," which issued on February 18, 1997. See Fig. 2d of the '293 patent.

The amino acid sequence of bFGF-1 is disclosed in U.S. Patent 5.604,293 (Fiddes) at Fig. 1b, as is a method for its expression. The mature forms of both hFGF-1 and bFGF-1 have 140 amino acid residues, bFGF-1 differs from hFGF-1 at 19 residue positions: 5 Pro to Leu, 21 His to Tyr, 31 Tyr to Val, 35 Arg to Lys, 40 Gin to Gly, 45 Gin to Phe, 47 Ser to Cys, 51 Tyr to Ile, 54 Tyr to Val, 64 Tyr to Phe, 80 Asn to Asp. 106 Asn to His, 109 Tyr to Val, 116 Ser to Arg, 117 Cys to Ser, 119 Arg to Leu, 120 Gly to Glu, 125 Tyr to Phe and 137 Tyr to Val. In most instances, the differences are conserved. Further, the differences at residue positions 116 and 119 merely interchange the position of the Arg.

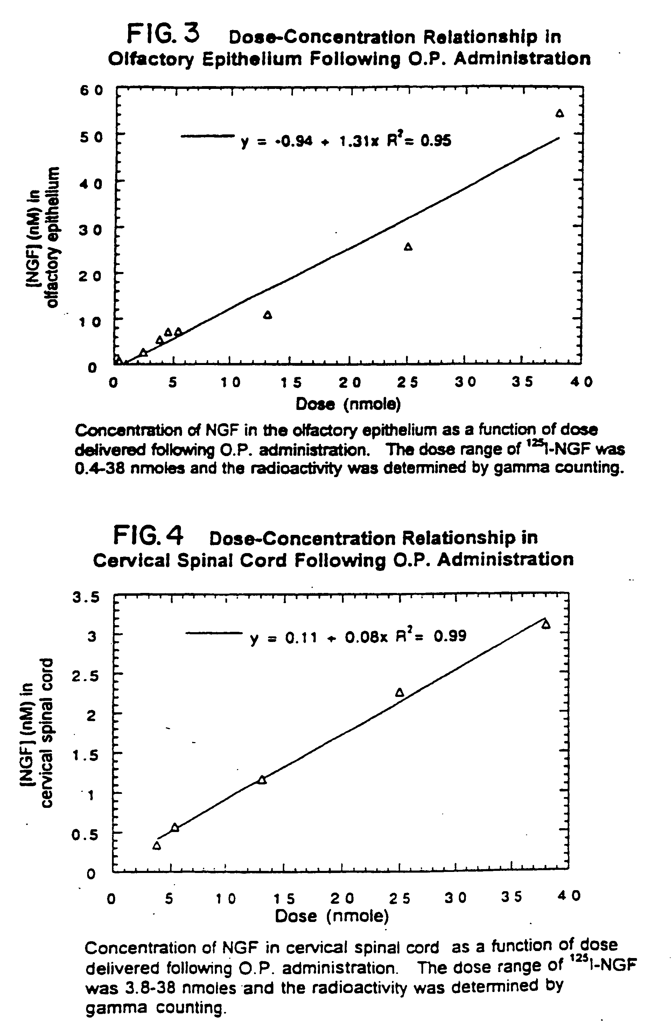

FGF-2: The amino acid sequence of human FGF-2 (hFGF-2) and methods for its recombinant expression are disclosed in U.S. Patent 5,439,818 (Fiddes) entitled "DNA Encoding Human Recombinant Basic Fibroblast Growth Factor," which issued on August 8. 1995 (see Fig. 4 therein). The amino acid sequence of bovine FGF-2 (bFGF-2) and various methods for its recombinant expression are disclosed in U.S. Patent 5,155,214, entitled "Basic Fibroblast Growth Factor," which issued on October 13. 1992. When the 146 residue forms of hFGF-2 and bFGF-2 are compared, their amino acid sequences are nearly identical with only two residues that differ. In particular, in going from hFGF-2 to bFGF-2, the sole differences occur at residue positions 112(Thr to Ser) and 128(Ser to Pro).

FGF-3: FGF-3 was first identified as an expression product of a mouse int-2 mammary tumor and its amino acid sequence is disclosed in Dickson et al., "Potential Oncogene Product Related to Growth Factors," Nature 326:833 (April 30, 1987). FGF-3 which has 243 residues when the N-terminal Met is excluded, is substantially longer than both FGF-2 (human and bovine) and FGF-2 (human and bovine). A comparison of amino acid residues for mFGF-3 relative to bFGF-1 and bFGF-2 is presented in overlap fashion in Dickson, et al. (1987). When the amino acid sequence of mFGF-3 is compared to bFGF-1 and bFGF-², FGF-3 has 5 locations containing residue inserts relative to both FGF-1 and FGF-2. The most significant of these inserts is a 12 and a 14 residue insert relative to FGF-2 and FGF-1, respectively, beginning at residue position 135 of FGF-3. Allowing for the inserts, Dickson discloses the mFGF-3 has 53 residue identities relative to FGF-1 and 69 residue identifies relative to FGF-2. In addition, FGF-3 contains a hydrophobic N-terminal extension of 10 residues relative to the N-terminus of the signal sequence in both FGF-1 and FGF-2. Relative to the C-terminus of bFGF-1 and bFGF-2, mFGF-3 contains an approximately 60 residue extension. It is unlikely that the C-terminal extension of mFGF-3 is necessary for activity. More likely, it is a moderator of activity by conferring receptor specificity on the FGF.

FGF-4: The amino acid sequence for the hst protein, now known as hFGF-4, was first disclosed by Yoshida, et al., "Genomic Sequence of hst, a Transforming Gene Enclosing a Protein Homologous to Fibroblast Growth Factors and the int-2 Enclosed Protein," PHAS USA, 84:7305-7309 (Oct. 1987) at Fig. 3. Including its leader sequence, hFGF-4 has 206 amino acid residues. When the amino acid sequences of hFGF-4, hFGF-1, hFGF-2 and mFGF-3 are compared, residues 72-204 of hFGF-4 have 43% homology to hFGF-2; residues 79-204 have 38% homology to hFGF-1; and residues 72-174 have 40% homology to mFGF-3. A comparison of these four sequences in overlap form is shown in Yoshida (1987) at Figure 3. Further, the Cys at residue positions 88 and 155 of hFGF-4 are highly conserved among hFGF-1, hFGF-2, mFGF-3 and hFGF-4 and are found in a homologous region.

The two putative cell binding sites of hFGF-2 occur at residue positions 36-39 and 77-81 thereof. See Yoshida (1987) at Fig. 3. The two putative heparin binding sites of hFGF-2 occur at residue positions 18-22 and 107-111 thereof. See Yoshida (1987) at Fig. 3. Given the substantial similarity between the amino acid sequences for human and bovine FGF-2, we would expect the cell binding sites for bFGF-2 to also be at residue positions 36-39 and 77-81 thereof, and the heparin binding sites to be at residue positions 18-22 and 107-111 thereof. In relation to hFGF-1, the putative cell binding sites occur at residues 27-30 and 69-72, and the putative heparin binding sites occur at residues 9-13 and 98-102. Insofar as mature bFGF-1 has the identical amino acids at residue positions 9-13, 27-30, 69-72 and 98-102 as does mature hFGF-2, bFGF-1 would be expected to have the same cell and heparin binding sites as does hFGF-1.

FGF-5: The cDNA and deduced amino acid sequences for hFGF-5 are disclosed in Zhan, et al., "The Human FGF-5 Oncogene Encodes a Novel Protein Related to Fibroblast Growth Factors,'' Molec. And Cell. Biol., 8(8):3487-3495 (Aug. 1988) at Fig. 1. Zhan also discloses a method for cloning hFGF-5. Another hFGF-5 has an amino acid sequence which differs from Zhan's sequence at residue position 236 (having a Lys instead of the Zhan's Asn) and at residue position 243 (having a Pro instead of Zhan's Ser). Both amino acid sequences for hFGF-5 have 266 amino acid residues that include a leader sequence of 67 residues upstream of the first residue of mature FGF-2 and a tail sequence that extends about 47 residues beyond the C-terminus of hFGF-2. A comparison between the amino acid sequences of hFGF-1. hFGF-2, mFGF-3, hFGF-4 and FGF-5 is presented in Fig. 2 of Zhan (1988). In Fig. 2 of Zhan, hFGF-1, hFGF-2, mFGRF-3 and hFGF-4 are identified as aFGF (i.e., acidic FGF), bFGF (i.e., basic FGF), int-2, and hstKS3, respectively, i.e., by their original names. In the above referenced comparison, two blocks of FGF-5 amino acid residues (90 to 180 and 187-207) showed substantial homology to FGF 1-4, i.e., 50.4% with FGF-4, 47.5% with FGF-3, 43.4% with FGF-2 and 40.2% with hFGF-1. See Zhan (1988) at Fig. 2 U.S. Patents 5,155,217 (Goldfarb) and 5,238,916 (Goldfarb), which correspond to the Zhan publication, refer to the FGF-5 of Zhan as FGF-3. However, the art (as evidenced by Coulier below) has come to recognize that the hFGF of Zhan (and of the Goldfarb patents) as FGF-5 and not as FGF-3. The two Goldfarb patents contain the same amino acid sequence for hFGF-5 as reported above by Zhan.

FGF-6: The cDNA and deduced amino acid sequence for hFGF-6 are disclosed in Coulier et al., "Putative Structure of the FGF-6 Gene Product and Role of the Signal Peptide," Oncogene 6:1437-1444 (1991) at Fig. 2. Coulier also discloses a method for cloning FGF-6. hFGF-6 is one of the largest of the FGFs, having 208 amino acid residues. When the amino acid sequences of human FGF-1, FGF-2, FGF-3, FGF-4, FGF-5, FGF-6 and FGF-7 are compared, there are strong similarities in the C-terminal two-thirds of the molecules (corresponding e.g., to residues 78-208 of hFGF-6. In particular, 23 residues of FGF-6, including the two cysteines at residue positions 90-157 of hFGF-6 were identical between the seven members of the family. This number increases to 33 residues when conserved amino acid residues are considered. The overall similarities between these seven human FGFs ranged from 32% to 70% identical residues and 48% to 79% conserved residues for the C-terminal two-thirds of the molecules. The sequence comparisons of hFGF-1 to hFGF-5 and hFGF-7, relative to hfGF-6, are shown in the FGF Table herein.

**FGF TABLE**

| **Amino Acid Sequence Comparison of hFGF-6 With Other hFGFs** | | | | |
|---|---|---|---|---|
| | Identical Residues* | Conserved Residues** | Identical Residues* (%) | Conserved Residues** (%) |
| hFGF-4 | 91 | 103 | 70 | 79 |
| hFGF-5 | 64 | 82 | 49 | 63 |
| hFGF-3 | 55 | 78 | 42 | 60 |
| hFGF-2 | 54 | 69 | 42 | 53 |
| hFGF-7 | 47 | 68 | 36 | 52 |
| hFGF-1 | 42 | 62 | 32 | 48 |

| | | | | |
|---|---|---|---|---|
| * Number and percentages of identical or conserved residues were calculated for the C-terininal two-thirds of the hFGF6 molecule (residues 78-208). | | | | |
| ** Conserved residues are defined according to the structure-genetic matrix of Feng et al., J. Mol. Evol., 21:112-125 (1985). | | | | |

Referring to the FGF Table, FGF-6 has the highest correspondence (91 identical residues/ 10o3 conserved residues) with FGF-4. This amounts to 70% identical and 79% conserved residues. HFGF-6 differed most from hFGF-3, hFGF-2, hFGF-7 and hFGF-1. with 42, 42, 36 and 32; identical residues, respectively.

An overlaid comparison of the amino acid sequences of FGFs 1-7 is shown in Figure 3 of incorporated Coulier (1991). Figure 3 of Coulier shows that when in the C-terminal two-thirds of the FGF molecules are aligned, there are 23 residue positions wherein the residues from all seven FGF members are identical. There are also ten residue positions wherein residues from all seven FGF members are conserved. Coulier (1991) at Figure 3. In combination, these identical and conserved residues form about 6 locations of three to five residues on the terminal two-thirds of each of the FGFs 1-7, wherein three to five residues are grouped together in all seven species of human FGF (i.e, hFGF 1-7).

FGF-7: The amino acid sequence of hFGF-7 is well-known in the art and disclosed in Miyamoto, et.al., "Molecular Cloning of a Novel Cyokine cDNA Encoding the Ninth Member of the Fibroblast Growth Factor Family, Which has a Unique Secretion Property.'' Mol. And Cell. Biol. 13(7):4251-4259 (1993) at Fig. 2. In Miyamoto, the hFGF-7 was referred to by its older name "KGF". FGF-7 has 191 amino acid sequences of hFGF-106 and hFGF-9 shows that the carboxy terminal two thirds of the FGF-7 has comparable homology with the distal two thirds of the other members of the group. See Miyamoto (1993) at page 4254 (Fig. 2).

FGF-8: The cDNA and deduced amino acid sequence of mFGRF-8 is well-known in the art and disclosed in Tanaka et. A., "Cloning and Characterization of an Androgen-Induced Growth Factor Essential for the Growth of Mouse Mammary Caricnoma Cells," PNAS USA, 89:8928-8932 (1992) at Fig. 2. Tanaka also discloses a method for making recombinant FGF-8. The mFGF-8 of Tanaka has 215 amino acid residues. MacArthur, et al., "FGF-8 isoforms activate receptor splice forms that are expressed in mesenchymal regions of mouse development," Development. 1212:3603-3613 (1995) discloses the FGF-8 has 8 different insoforms that differ at the mature N-terminus but that are identical over the C-terminal region. The 8 isoforms arise because FGF-8 has 6 exons of which the first four (which correspond to the first exon of most other FGF genes) result in alternative splicing.

FGF-9: The cDNA and deduced amino acid sequences of human and murine FGF-9 are known in the art and methods for their recombinant expressions are disclosed in Santos-Ocamp, et. Al., "Expression and Biological Activity of Mouse Fibroblast Growth Factor," J. Biol. Chem., 271(3):1726-1731 (1996). Both the human and murine FGF-9 molecules have 208 amino acid residues and sequences that differ by only two residues. In particular, hFGF-9 has Ser and Asn at residues 9 and 34, respectively. FGF-9 has complete preservation of the conserved amino acids that define the FGF family. Santos-Ocamp (1996) at page 1726. Half-maximal activation of FGf-9 is seen at 185 ng/ml heparin, whereas half-maximal activation of FGF-1 is seen at 670 ng/ml heparin. Santos-Ocampo (1996) at page 1730. When compared to FGF-1, both FGF-1, both FGF-2 and FGF-9 require lower heparin concentrations for optimal activity.

FGF-98: The cDNA and amino acid sequence of hFGF-98 and a method for its recombinant expression are disclosed in provisional patent application Serial No. 60/083,553 which is hereby incorporated herein by reference in its entirety. HFGF-98, which is also known as hFGF-18, has 207 amino acid residues. Thus, hFGF-6 (207 residues), hFGF-9 (208 residues) and hFGF-98 (207 residues) are similar in size.

bFGF-2, and other FGFs, can be made as described in U.S. Patent 5,155.214 ("the '214 patent"). The recombinant bFGF-2, and other FGFs, can be purified to pharmaceutical quality (98% or greater purity) using the techniques described in detail in U.S. Pat. 4,956,455 (the '455 patent), entitled "Bovine Fibroblast Growth Factor" which issued on 09/11/90.

Biologically active variants of FGF are also encompassed by the method of the present invention. Such variants should retain FGF activities, particularly the ability to bind to FGF receptor sites. FGF activity may be measured using standard FGF bioassays, which are known to those of skill in the art. Representative assays include known radioreceptor assays using membranes, a bioassay that measures the ability of the molecule to enhance incorporation of tritiated thymidine, in a dose-dependent manner, into the DNA of cells, and the like. Preferably, the variant has at least the same activity as the native molecule.

In addition to the above described FGFs, the neurotrophic agent also includes an active fragment of any one of the above-described FGFs. In its simplest form, the active fragment is made by the removal of the N-terminal methionine, using well-known techniques for N-terminal Met removal, such as a treatment with a methionine aminopeptidase. A second desirable truncation includes an FGF without its leader sequence. Those skilled in the art recognize the leader sequence as the series of hydrophobic residues at the N-terminus of a protein that facilitate its passage through a cell membrane but that are not necessary for activity and that are not found on the mature protein.

Preferred truncations on the FGFs are determined relative to mature hFGF-2 (or the analogous bFGF-2) having 146 residues. As a general rule, the amino acid sequence of an FGF is aligned with FGF-2 to obtain maximum homology. Portions of the FGF that extend beyond the corresponding N-terminus of the aligned FGF-2 are generally suitable for deletion without adverse effect. Likewise, portions of the FGF that extend beyond the C-terminus of the aligned FGF-2 are also capable of being deleted without adverse effect.

Fragments of FGF that are smaller than those described can also be employed in the present invention, so long as they retain the cell binding portions of FGF and at least one heparin binding segment. In the case of mature FGF-2 having residues 1-146, the two putative cell binding sites occur at residue positions 36-39 and 77-81 thereof. See Yoshida, et al., "Genomic Sequence of hst, a Transforming Gene Encoding a Protein Homologous to Fibroblast Growth Factors and the int-2-Encoded Protein," PNAS USA, 84:7305-7309 (Oct. 1987) at Fig. 3. The two putative heparin binding sites of hFGF-2 occur at residue positions 18-22 and 107-11 thereof. See, Yoshida (1987) at Fig. 3. Accordingly, the active fragments of an FGF typically encompass those terminally truncated fragments of an FGF that when aligned to mature FGF-2 (having residues 1-146) to maximize homology, have at least residues that correspond to residue positions 30-110 of FGF-2; more typically, at least residues that correspond to residues 18-146 of FGF-2.

Suitable biologically active variants can be FGF analogues or derivatives. By "analogue" is intended an analogue of either FGF or an FGF fragment that includes a native FGF sequence and structure having one or more amino acid substitutions, insertions, or deletions. Analogs having one or more peptoid sequences (peptide mimic sequences) are also included (see e.g. International Publication No. WO 91/04282). By "derivative" is intended any suitable modification of FGF, FGF fragments, or their respective analogues, such as glycosylation, phosphorylation, or other addition of foreign moieties, so long as the FGF activity is retained. Methods for making FGF fragments, analogues, and derivatives are available in the art.

In addition to the above described FGFs, the method of the present invention can also employ an active mutein or variant thereof. By the term active mutein, as used in conjunction with an FGF, is meant a mutated form of the naturally occurring FGF.FGF muteins or variants will have at least 70%, preferably 80%, more preferably 85%, even more preferably 90% to 95% or more, and most preferably 98% or more amino acid sequence identity to the amino acid sequence of the reference FGF molecule. A mutein or variant may, for example, differ by as few as 1 to 10 amino acid residues, such as 6-10, as few as 5, as few as 4, 3, 2, or even 1 amino acid residue.

The sequence identity can be determined as described hereinabove. For FGF, a preferred method for determining sequence identify employs the Smith-Waterman homology search algorithm *(Meth. Mol. Biol.* 70:173-187 (1997)) as implemented in MSPRCH program (Oxford Molecular) using an affine gap search with the following search parameters: gap open penalty of 12. and gap extension penalty of 1. Preferably, the mutations are "conservative amino acid substitutions" using L-amino acids, wherein one amino acid is replaced by another biologically similar amino acid. As previously noted, conservative amino acid substitutions are those that preserve the general charge, hydrophobicity, hydrophilicity, and/or steric bulk of the amino acid being substituted. Examples of conservative substitutions are those between the following groups: Gly/Ala, Val/Ile/Leu, Lys/Arg, Asn/Gln, Glu/Asp, Ser/Cys/Thr, and Phe/Trp/Tyr. In the case of FGF-2, an example of such a conservative amino acid substitution includes the substitution of serine for one or both of the cysteines at residue positions which are not involved in disulfide formation, such as residues 87 and 92 in mature FGF-2 (having residues 1-146).

One skilled in the art, using art known techniques, is able to make one or more point mutations in the DNA encoding any of the FGFs to obtain expression of an FGF polypeptide mutein (or fragment mutein) having angiogenic activity for use in method of the present invention. To prepare an biologically active mutein of an FGF, one uses standard techniques for site directed mutagenesis, as known in the art and/or as taught in Gilman, *et al.,* Gene, 8:81 (1979) or Roberts, *et al.,* Nature, 328:731 (1987), to introduce one or more point mutations into the cDNA that encodes the FGF.

### NGF

The term "NGF" as used herein refers to nerve growth factor (NGF). NGF was originally isolated as a complex of molecular weight 130 kDa and a sedimentation coefficient of 7S. This 7S complex included three types of subunits, the "β" subunit carrying all of the biological activities of NGF. The term β-NGF can be used to mean NGF, and the term NGF typically refers to β-NGF. NGF is a dimer of two identical peptide chains each having 118 amino acids and a molecular weight of about 26.5 kDa. Nerve growth factor stimulates mitosis and growth processes associated with cell, particularly nerve cell, development.

In one embodiment of the invention, increasing in the amount of NGF to a therapeutically effective level is achieved via administration of a pharmaceutical composition including a therapeutically effective dose. The NGF to be administered can be from any animal species including, but not limited to, rodent, avian, canine, bovine, porcine, equine, and, preferably, human. Preferably the NGF is from a mammalian species, and more preferably is from a mammal of the same species as the mammal undergoing treatment.

Biologically active variants of NGF may also be used in the present invention. Such variants should retain NGF activities, particularly the ability to bind to NGF receptor sites. NGF activity may be measured using standard NGF bioassays, which are known to those of skill in the art. Representative assays include known radioreceptor assays using membranes, a bioassay that measures the ability of the molecule to enhance incorporation of tritiated thymidine, in a dose-dependent manner, into the DNA of cells, and the like. The biological activities of NGF include increasing levels of choline acetyl transferase. Preferably, the variant has at least the same activity as the native molecule.

Suitable biologically active variants can be NGF fragments, analogues, and derivatives. By "NGF fragment" is intended a protein consisting of only a part of the intact NGF sequence and structure, and can be a C-terminal deletion or N-terminal deletion of NGF. By "analogue" is intended an analogue of either NGF or an NGF fragment that includes a native NGF sequence and structure having one or more amino acid substitutions, insertions, or deletions. Analogs having one or more peptoid sequences (peptide mimic sequences) are also included (see e.g. International Publication No. WO 91/04282). By "derivative" is intended any suitable modification of NGF, NGF fragments, or their respective analogues, such as glycosylation, phosphorylation, or other addition of foreign moieties, so long as the NGF activity is retained. Methods for making NGF fragments, analogues, and derivatives are available in the art.

NGF variants will have at least 70%, preferably 80%, more preferably 85%, even more preferably 90% to 95% or more, and most preferably 98% or more amino acid sequence identity to the amino acid sequence of the reference NGF molecule. A variant may, for example, differ by as few as I to 10 amino acid residues, such as 6-10, as few as 5, as few as 4, 3, 2, or even 1 amino acid residue. Sequence identity and alignment can be determined as described hereinabove.

The art provides substantial guidance regarding the preparation and use of NGF variants. A fragment of NGF will generally include at least about 10 contiguous amino acid residues of the full-length molecule, preferably about 15-25 contiguous amino acid residues of the full-length molecule, and most preferably about 20-50 or more contiguous amino acid residues of full-length NGF.

The NGF used in the present invention can be in its substantially purified, native, recombinantly produced, or chemically synthesized forms. NGF can be isolated and purified from serum, plasma or other tissues by methods known in the art. NGF can also be chemically synthesized by the solid phase method (see Li *et al.* (1983) *Proc. Natl. Acad. Sci. USA* 80: 2216-2220).

Genetic engineering by recombinant DNA techniques can be the most efficient way of producing NGF. The human DNA sequence encoding NGF is known and can be introduced into host cells for expression. NGF can be produced by recombinant DNA techniques in E. *coli,* yeast, insect, and mammalian cells. Secreted NGF can be made by adding a signal sequence to the DNA sequence encoding NGF. In addition, the DNA sequence encoding NGF can be manipulated to make NGF fragments, analogues, or derivatives. Such recombinant DNA techniques are generally available in the art. See, for example, International Publication No. WO 96/07424.

### Pharmaceutical Composition

Increases in the amount of neurotrophic agent in the CNS, brain, and/or spinal cord to a therapeutically effective level may be obtained via administration of a pharmaceutical composition including a therapeutically effective dose of this agent. By "therapeutically effective dose" is intended a dose of neurotrophic agent that achieves the desired goal of increasing the amount of this agent in a relevant portion of the CNS, brain, and/or spinal cord to a therapeutically effective level enabling a desired biological activity of the agent. Desired biological activities include an increase in protein phosphorylation, particularly of the IGF-I receptor, in response to IGF-I: and an increase in acetylcholine acetyl transferase in response to NGF.

The invention is, in particular, directed to use in the manufacture of a medicament on composition that can be employed for delivery of a neurotrophic agent to the CNS, brain, and/or spinal cord upon administration to the nasal cavity. The composition can include, for example, any pharmaceutically acceptable additive, carrier, or adjuvant that is suitable for administering a neurotrophic agent through the mucosa or epithelium of the nasal cavity. Preferably, the pharmaceutical composition can be employed in diagnosis, prevention, or treatment of a disease, disorder, or injury of the CNS, brain, and/or spinal cord. Preferably, the composition includes a neurotrophic agent in combination with a pharmaceutical carrier, additive, and/or adjuvant that can promote the transfer of the neurotrophic agent within or through the mucosa or epithelium of the nasal cavity, or along or through a neural system. Alternatively, the neurotrophic agent may be combined with substances that may assist in transporting the neurotrophic agent to sites of nerve cell damage. The composition can include one or several neurotrophic agents.

The composition typically contains a pharmaceutically acceptable carrier mixed with the neurotrophic agent and other components in the pharmaceutical composition. By "pharmaceutically acceptable carrier'' is intended a carrier that is conventionally used in the art to facilitate the storage, administration, and/or the healing effect of the neurotrophic agent. A carrier may also reduce any undesirable side effects of the neurotrophic agent. A suitable carrier should be stable, i.e., incapable of reacting with other ingredients in the formulation. It should not produce significant local or systemic adverse effect in recipients at the dosages and concentrations employed for treatment. Such carriers are generally known in the art.

Suitable carriers for this invention include those conventionally used for large stable macromolecules such as albumin, gelatin, collagen, polysaccharide, monosaccharides, polyvinylpyrrolidone, polylactic acid, polyglycolic acid, polymeric amino acids, fixed oils, ethyl oleate, liposomes, glucose, sucrose, lactose, mannose, dextrose, dextran, cellulose, mannitol, sorbitol and polyethylene glycol (PEG).

Water, saline, aqueous dextrose, and glycols are preferred liquid carriers, particularly (when isotonic) for solutions. The carrier can be selected from various oils, including those of petroleum, animal, vegetable or synthetic origin, for example, peanut oil, soybean oil, mineral oil, sesame oil, and the like. Suitable pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk, glycerol, propylene glycol, water and ethanol. The compositions can be subjected to conventional pharmaceutical expedients, such as sterilization, and can contain conventional pharmaceutical additives, such as preservatives, stabilizing agents, wetting, or emulsifying agents, salts for adjusting osmotic pressure and buffers.

Other acceptable components in the composition include, but are not limited to, buffers that enhance isotonicity such as water, saline, phosphate, citrate, succinate, acetic acid, and other organic acids or their salts. Typically, the pharmaceutically acceptable carrier also includes one or more stabilizers, reducing agents, anti-oxidants and/or anti-oxidant chelating agents. The use of buffers, stabilizers, reducing agents, anti-oxidants and chelating agents in the preparation of protein based compositions, particularly pharmaceutical compositions, is well-known in the art. *See,* Wang *et al., "Review of Excipients und pHs for Parenteral Products Used in the United States." J. Parent. Drug Assn.,* 34(6):452-462 (1980); Wang *et al., "Paremeral Formulations of Proteins and Peptides: Stability and Stabilizers," J. Parent. Sci. and Tech.,* 42:S4-S26 (Supplement 1988); Lachman, *et al.,* *"Antioxidants and Chelating Agents as Stabilizers in Liquid Dosage Forms- Part 1," Drug and Cosmetic Industry,* 102(1): 36-38, 40 and 146-148 (1968): Akers, M.J., *"Antioxidants in Pharmaceutical Producls, " J. Parent. Sci. and Tech.,* 36(5):222-228 (1988); and Methods in Enzymology, Vol. XXV, Colowick and Kaplan eds., *"Reduction of Disulfide Bonds in Proteins with Dithiothreitol,* " by Konigsberg, pages 185-188.

Suitable buffers include acetate, adipate, benzoate, citrate. lactate, maleate, phosphate, tartarate, borate, tri(hydroxymethyl aminomethane), succinate, glycine, histidine, the salts of various amino acids, or the like, or combinations thereof. *See* Wang (1980) at page 455. Suitable salts and isotonicifiers include sodium chloride, dextrose, mannitol, sucrose, trehalose, or the like. Where the carrier is a liquid, it is preferred that the carrier is hypotonic or isotonic with oral, conjunctival or dermal fluids and have a pH within the range of 4.5-8.5. Where the carrier is in powdered form, it is preferred that the carrier is also within an acceptable non-toxic pH range.

Suitable reducing agents, which maintain the reduction of reduced cysteines, include dithiothreitol (DTT also known as Cleland's reagent) or dithioerythritol at 0.01% to 0.1% wt/wt; acetylcysteine or cysteine at 0.1% to 0.5% (pH 2-3); and thioglycerol at 0.1% to 0.5% (pH 3.5 to 7.0) and glutathione. *See* Akers (1988) at pages 225 to 226. Suitable antioxidants include sodium bisulfite, sodium sulfite, sodium metabisulfite, sodium thiosulfate, sodium formaldehyde sulfoxylate, and ascorbic acid. *See* Akers (1988) at pages 225. Suitable chelating agents, which chelate trace metals to prevent the trace metal catalyzed oxidation of reduced cysteines, include citrate, tartarate, ethylenediaminetetraacetic acid (EDTA) in its disodium, tetrasodium, and calcium disodium salts, and diethylenetriamine pentaacetic acid (DTPA). *See e.g.,* Wang (1980) at pages 457-458 and 460-461, and Akers (1988) at pages 224-227.

The composition can include one or more preservatives such as phenol, cresol, paraaminobenzoic acid, BDSA, sorbitrate, chlorhexidine, benzalkonium chloride, or the like. Suitable stabilizers include carbohydrates such as threlose or glycerol. The composition can include a stabilizer such as one or more of microcrystalline cellulose, magnesium stearate, mannitol, or sucrose to stabilize, for example, the physical form of the composition; and one or more of glycine, arginine, hydrolyzed collagen, or protease inhibitors to stabilize, for example, the chemical structure of the composition. Suitable suspending agents include carboxymethyl cellulose, hydroxypropyl methylcellulose, hyaluronic acid, alginate, chonodroitin sulfate, dextran, maltodextrin, dextran sulfate, or the like. The composition can include an emulsifier such as polysorbate 20, polysorbate 80, pluronic, triolein, soybean oil, lecithins, squalene and squalanes, sorbitan treioleate, or the like. The composition can include an antimicrobial such as phenylethyl alcohol, phenol, cresol, benzalkonim chloride, phenoxyethanol, chlorhexidine, thimerosol, or the like. Suitable thickeners include natural polysaccharides such as mannans, arabinans, alginate, hyaluronic acid, dextrose, or the like; and synthetic ones like the PEG hydrogels of low molecular weight; and aforementioned suspending agents.

The composition can include an adjuvant such as cetyl trimethyl ammonium bromide, BDSA, cholate, deoxycholate, polysorbate 20 and 80, fusidic acid, and in the case of DNA delivery, preferably, a cationic lipid. Suitable sugars include glycerol, threose, glucose, galactose and mannitol, sorbitol. A suitable protein is human serum albumin.

Preferred compositions include one or more of a solubility enhancing additive, preferably a cyclodextrin; a hydrophilic additive, preferably a mono or oligosachharide; an absorption promoting additives, preferably a cholate, a deoxycholate, a fusidic acid, or a chitosan; a cationic surfactant, preferably a cetyl trimethyl ammonium bromide; a viscosity enhancing additive, preferably to promote residence time of the composition at the site of administration, preferably a carboxymethyl cellulose, a maltodextrin, an alginic acid, a hyaluronic acid, or a chondroitin sulfate; or a sustained release matrix, preferably a polyanhydride, a polyorthoester, a hydrogel, a particulate slow release depo system, preferably a polylactide co-glycolides (PLG), a depo foam, a starch microsphere, or a cellulose derived buccal system; a lipid based carrier, preferably an emulsion, a liposome, a niosome, or a micelle. The composition can include a bilayer destabilizing additive, preferably a phosphatidyl ethanolamine; a fusogenic additive, preferably a cholesterol hemisuccinate.

These lists of carriers and additives is by no means complete and a worker skilled in the art can choose excipients from the GRAS (generally regarded as safe) list of chemicals allowed in the pharmaceutical preparations and those that are currently allowed in topical and parenteral formulations.

For the purposes of this invention, the pharmaceutical composition including neurotrophic agent can be formulated in a unit dosage and in a form such as a solution, suspension, or emulsion. The neurotrophic agent may be administered to the nasal cavity as a powder, a granule, a solution, a cream, a spray (e.g., an aerosol), a gel, an ointment, an infusion, an injection, a drop, or a sustained release composition, such as a polymer disk. Other forms of compositions for administration include a suspension of a particulate, such as an emulsion, a liposome, an insert that releases the neurotrophic agent slowly, and the like. The powder or granular forms of the pharmaceutical composition may be combined with a solution and with a diluting, dispersing or surface active neurotrophic agent. Additional preferred compositions for administration include a bioadhesive to retain the neurotrophic agent at the site of administration; a spray, paint, or swab applied to the mucosa or epithelium; The composition can also be in the form of lyophilized powder, Which can be converted into solution, suspension, or emulsion before administration. The pharmaceutical composition having neurotrophic agent is preferably sterilized by membrane filtration and is stored in unit-dose or multi-dose containers such as sealed vials or ampoules.

The method for formulating a pharmaceutical composition is generally known in the art. A thorough discussion of formulation and selection of pharmaceutically acceptable carriers, stabilizers, and isomolytes can be found in *Remington's Pharmaceutical Sciences* (18^{th} ed.; Mack Publishing Company, Eaton, Pennsylvania, 1990).

The neurotrophic agent of the present invention can also be formulated in a sustained-release form to prolong the presence of the pharmaceutically active neurotrophic agent in the treated mammal, generally for longer than one day. Many methods of preparation of a sustained-release formulation are known in the art and are disclosed in *Remington's Pharmaceutical Sciences* (18^{th} ed.; Mack Publishing Company, Eaton, Pennsylvania, 1990).

Generally, the neurotrophic agent can be entrapped in semipermeable matrices of solid hydrophobic polymers. The matrices can be shaped into films or microcapsules. Examples of such matrices include, but are not limited to, polyesters, copolymers of L-glutamic acid and gamma ethyl-L-glutamate (Sidman *et al.* (1983) *Biopolymers* 22: 547-556), polylactides (U.S. Patent No. 3,773,919 and EP 58,481), polylactate polyglycolate (PLGA) such as polylactide-co-glycolide (see, for example, U.S. Patent Nos. 4,767,628 and 5,654,008), hydrogels (see, for example, Langer *et al.* (1981) *J*. *Biomed. Mater. Res.* 15: 167-277; Langer (1982) *Chem. Tech.* 12: 98-105), non-degradable ethylene-vinyl acetate (e.g. ethylene vinyl acetate disks and poly(ethylene-co-vinyl acetate)), degradable lactic acid-glycolic acid copolyers such as the Lupron Depot™, poly-D-(-)-3-hydroxybutyric acid (EP 133.988), hyaluronic acid gels (see, for example, U.S. Patent 4,636,524) and alginic acid suspensions.

Suitable microcapsules can also include hydroxymethylcellulose or gelatin-microcapsules and polymethyl methacrylate microcapsules prepared by coacervation techniques or by interfacial polymerization. See the copending application entitled *"Method for Producing Sustained-release Formulations,"* U.S. Patent Application Serial No. 09/187,780, filed November 6, 1998, wherein a neurotrophic agent is encapsulated in PLGA microspheres. In addition, microemulsions or colloidal drug delivery systems such as liposomes and albumin microspheres, may also be used. See *Remington's Pharmaceutical Sciences* (18^{th} ed.; Mack Publishing Company Co., Eaton, Pennsylvania. 1990). Other sustained release compositions employ a bioadhesive to retain the neurotrophic agent at the site of administration.

Among the optional substances that may be combined with the neurotrophic agent in the pharmaceutical composition are lipophilic substances that can enhance absorption of the neurotrophic agent through the mucosa or epithelium of the nasal cavity to damaged nerve cells in the CNS. The neurotrophic agent may be mixed with a lipophilic adjuvant alone or in combination with a carrier, or may be combined with one or several types of micelle or liposome substances. Among the preferred lipophilic substances are cationic liposomes including one or more of phosphatidyl choline, lipofectin, DOTAP, or the like. These liposomes may include other lipophilic substances such as gangliosides and phosphatidylserine (PS). Also preferred are micellar additives such as GM-1 gangliosides and phosphatidylserine (PS), which may be combined with the neurotrophic agent either alone or in combination. GM-1 ganglioside can be included at 1-10 mole percent in any liposomal compositions or in higher amounts in micellar structures. Protein agents can be either encapsulated in particulate structures or incorporated as part of the hydrophobic portion of the structure depending on the hydrophobicity of the protein agent. One preferred liposomal formulation employs Depofoam. A neurotrophic agent can be encapsulated in multivesicular liposomes, as disclosed in the copending application entitled *"High and Low Load Formulations of IGF-I in Multivesicular Liposomes,"* U.S. Patent Application Serial No. 08/923.531, filed September 8, 1997.

When the neurotrophic agent is an FGF and the pharmaceutically acceptable carrier is a liquid carrier, a typical pharmaceutical composition can include about 50 to about 10.000 ng/ml, more typically about 50 to 1500 ng/ml, of an FGF or an active fragment or mutein thereof 10 mM thioglycerol. 135 mM NaCl, 10 mM sodium citrate, and 1mM EDTA, pH 5. A suitable diluent or flushing agent for the above described composition is any of the above described carriers. Typically, the diluent is the carrier solution itself, which in this example includes 10 mM thioglycerol. 135 mM NaCl, 10 mM sodium citrate and 1 mM EDTA, pH 5.

When provided in liquid form, such an FGF, or other neurotrophic agent, composition or unit dose can become unstable when stored for extended periods of time. To maximize stability and shelf life, the pharmaceutical compositions and the unit dose compositions should be stored frozen at -60°C. When thawed, the solution can stable for 6 months at refrigerated conditions. A typical vial of the pharmaceutical composition would include about 1.0 to 100 ml (more typically, about 1.0 to 25 ml; most typically, about 1.0 to 10 ml) of the above described pharmaceutically acceptable carrier containing therein from about 5 ng to about 10.000 ng of FGF, or another neurotrophic agent, or an active fragment or mutein thereof.

When the neurotrophic agent is IGF-I, or another neurotrophic agent, the pharmaceutical composition may additionally include a solubilizing compound. For IGF-I, a preferred solubilizing agent, includes a guanidinium group and that is capable of enhancing the solubility of a neurotrophic agent like IGF-I. Examples of such solubilizing compounds include the amino acid arginine, as well as amino acid analogs of arginine that retain the ability to enhance solubility of a neurotrophic agent at pH 5.5 or greater. Such analogs include, without limitation, dipeptides and tripeptides that contain arginine. By "enhancing the solubility" of a neurotrophic agent is intended increasing the amount of neurotrophic agent that can be dissolved in solution at pH 5.5 or greater in the presence of a guanidinium-containing compound compared to the amount of neurotrophic agent that can be dissolved at pH 5.5 or greater in a solution with the same components but lacking the guanidinium-containing compound. The ability of a guanidinium-containing compound to enhance the solubility of a neurotrophic agent can be determined using methods well known in the art. In general, the concentration of the solubilizing compound present in the composition will be from about 10 mM to about 1 M, and, for example, in the case of the compound arginine, in a concentration range of about 20 mM to about 200 mM, as disclosed in the copending application entitled *"Compositions Providing for Increased IGF-I Solubility,"* U.S. Patent Application Serial No. 09/188,051, filed November 6, 1998.

A preferred embodiment of the present composition includes an effective amount of NGF with a pharmaceutically-acceptable liquid carrier containing an appropriate amount of micelles included of GM-1 ganglioside. GM-1 is thought to act synergistically with nerve growth factor (NGF) to protect neurons and promote nerve regeneration and repair. Another preferred embodiment includes an antisense oligonucleotide for treating brain tumors. Another preferred embodiment of the composition includes the combination of an effective amount of basic fibroblast growth factor (bFGF) or insulin like growth factor-I (IGF-I) with poly(ethylene-co-vinyl acetate) to provide for controlled release of the neurotrophic agents, for example for the treatment of stroke.

### Administering the Neurotrophic Agent

The neurotrophic agent is typically administered in a dose sufficient to provide a therapeutically effective level in the portion of the CNS, brain, and/or spinal cord that can benefit from the agent. A neurotrophic agent generally exhibits biological activity at a concentration in or surrounding a tissue of about 10⁻¹² M to about 10⁻⁹ M, preferably about 10⁻¹¹ M to about 10⁻⁹ M, preferably about 10⁻¹⁰ M. A few of the most potent neurotrophic agents (e.g. activity dependent neurotrophic factor, ADNF) exhibit their biological activity in a range as low as about 1O⁻¹⁵ M. Preferred neurotrophic agents, such as NGF, IGF-I, and bFGF exhibit biological effects in relevant tissues of the CNS, brain, and/or spinal cord at concentrations of about 10⁻¹¹ M to about 10⁻⁹ M.

These concentrations of neurotrophic agent can be achieved in relevant tissues of the CNS, brain, and/or spinal cord of a rat after nasal administration of a therapeutically effective dose of about 0.1 nmol to about 10 nmol. For example, NGF can be found at relevant concentrations in the rat CNS, brain, and/or spinal cord after administration of about 0.5 nmol to about 10 nmol of this agent and, it is believed, at lower concentrations. IGF-I and bFGF can be found at relevant concentrations in the rat CNS, brain, and/or spinal cord after administration of about 1 nmol to about 10 nmol of this agent, and, it is believed, at lower concentrations. In some regimens, therapeutically effective doses for administration of a neurotrophic agent to a rat include about 0.1, 0.2, 0.3, 0.4, 0.5. 0.6. 0.7, 0.8, 0.9, 1.0, 2, 3, 4, 5, 6, 7, 8, 9, or 10 nmol. These doses depend on factors including the efficiency with which the agent is transported from the nasal cavity to the brain. A larger total dose can be delivered by multiple administrations of agent.

Based on considerations including the relative size and mass of portions of the rat and human brains that are advantageous targets for delivery of a neurotrophic agent, therapeutically effective human doses of neurotrophic agent can range from about 1 nmol to about 1000 nmol. In particular. NGF, bFGF, and IGF-I can be administered to a human at a therapeutically effective dose of about 1 nmol to about 1000 nmol. In some regimens, therapeutically effective doses for administration of a neurotrophic agent to a human include about 1, 2, 3, 4, 5, 6, 7, 8, 9, 10, 20, 30, 40, 50, 60, 70, 80, 90, 100, 200, 300. 400. 500, 600, 700, 800, 900, or 1000 nmol. These doses depend on factors including the efficiency with which the agent is transported from the nasal cavity to the brain. A larger total dose can be delivered by multiple administrations of agent.

The pharmaceutical composition having a unit dose of neurotrophic agent can be, for example, in the form of solution, suspension, emulsion, or a sustained-release formulation. Preferably, the total volume of one dose of the pharmaceutical composition ranges from about 10 µl to about 0.2 ml, preferably from about 50 µl to about 200 µl. It is apparent that the suitable volume can vary with factors such as the size of the nasal cavity to which the agent is administered and the solubility of the components in the composition.

Such a therapeutically effective dose can deliver neurotrophic agent to a portion of the CNS, brain, or spinal cord relevant to treating a disease, disorder, or injury of these tissues. For example, delivering a neurotrophic agent to the olfactory bulbs, the hippocampal formation, and/or the frontal cortex can be beneficial to treating Alzheimer's disease. Similarly, delivering a neurotrophic agent to the midbrain, including the substantia nigra and locus ceruleus, and/or to the brainstem can be beneficial to treating Parkinson's disease. Movement disorders known as ataxias can benefit from treatment directed at the cerebellum. Stroke or injury can affect most parts of the CNS. brain, and/or spinal cord. The the invention can be used to deliver therapeutically effective amounts of a neurotrophic agent to portions of the brain and CNS including the olfactory bulbs, the hippocampal formation, the frontal cortex, the midbrain, the brainstem, and the spinal cord, which portions are relevant to several diseases or disorders of the CNS, brain, and/or spinal cord.

It is recognized that the total amount of neurotrophic agent administered as a unit dose to a particular tissue will depend upon the type of pharmaceutical composition being administered, that is whether the composition is in the form of, for example, a solution, a suspension, an emulsion, or a sustained-release formulation. For example, where the pharmaceutical composition comprising a therapeutically effective amount of neurotrophic agent is a sustained-release formulation, neurotrophic agent is administered at a higher concentration.

It should be apparent to a person skilled in the art that variations may be acceptable with respect to the therapeutically effective dose and frequency of the administration of neurotrophic agent in this embodiment of the invention. The amount of the neurotrophic agent administered will be inversely correlated with the frequency of administration. Hence, an increase in the concentration of neurotrophic agent in a single administered dose, or an increase in the mean residence time in the case of a sustained release form of neurotrophic agent, generally will be coupled with a decrease in the frequency of administration.

It is appreciated by those of skill in the art that the actual dose of the neurologic agent will depend on a variety of factors that may be specific to the subject undergoing dosing. These factors should be taken into consideration when determining the therapeutically effective dose of neurotrophic agent and frequency of its administration. For example, the effective dose can depend on the species, age, weight, or general health of the subject; the severity of the disease or disorder; the size and location of the portion of the brain in which an effective amount of agent must be achieved; the frequency and duration of dosing; the type of formulation administered; the characteristics, such as lipophilicity, of the agent and composition; the nature of the agent and its receptors, if any; and the like. Generally, a higher dosage is preferred if the disease or disorder is more severe. It is believed that the rate of transport through a neuron may be independent of species and agent.

Some minor degree of experimentation may be required to determine the most effective dose and frequency of dose administration, this being well within the capability of one skilled in the art once apprised of the present disclosure.

### Intermittent Dosing

In another embodiment of the invention, the pharmaceutical composition comprising the therapeutically effective dose of neurotrophic agent is administered intermittently. By "intermittent administration" is intended administration of a therapeutically effective dose of neurotrophic agent, followed by a time period of discontinuance, which is then followed by another administration of a therapeutically effective dose, and so forth. Administration of the therapeutically effective dose may be achieved in a continuous manner, as for example with a sustained-release formulation, or it may be achieved according to a desired daily dosage regimen, as for example with one, two, three or more administrations per day. By "time period of discontinuance" is intended a discontinuing of the continuous sustained-released or daily administration of neurotrophic agent. The time period of discontinuance may be longer or shorter than the period of continuous sustained-release or daily administration. During the time period of discontinuance, the neurotrophic agent level in the relevant tissue is substantially below the maximum level obtained during the treatment. The preferred length of the discontinuance period depends on the concentration of the effective dose and the form of neurotrophic agent used. The discontinuance period can be at least 2 days, preferably is at least 4 days, more preferably is at least I week and generally does not exceed a period of 4 weeks. When a sustained-release formulation is used, the discontinuance period must be extended to account for the greater residence time of neurotrophic agent at the site of injury. Alternatively, the frequency of administration of the effective dose of the sustained-release formulation can be decreased accordingly. An intermittent schedule of administration of neurotrophic agent can continue until the desired therapeutic effect, and ultimately treatment of the disease or disorder, is achieved.

In yet another embodiment, intermittent administration of the therapeutically effective dose of neurotrophic agent is cyclic. By "cyclic" is intended intermittent administration accompanied by breaks in the administration, with cycles ranging from about 1 month to about 2, 3, 4, 5, or 6 months, more preferably about 3 months to about 6 months. For example, the administration schedule might be intermittent administration of the effective dose of neurotrophic agent, wherein a single short-term dose is given once per week for 4 weeks, followed by a break in intermittent administration for a period of 3 months, followed by intermittent administration by administration of a single short-term dose given once per week for 4 weeks; followed by a break in intermittent administration for a period of 3 months, and so forth. As another example, a single short-term dose may be given once per week for 2 weeks, followed by a break in intermittent administration for a period of I month, followed by a single short-term dose given once per week for 2 weeks, followed by a break in intermittent administration for a period of 1 month, and so forth. A cyclic intermittent schedule of administration of neurotrophic agent to subject may continue until the desired therapeutic effect, and ultimately treatment of the disorder or disease, is achieved.

### Neuronal Transport

One embodiment of the present invention includes use in the manufacture of a medicament for use in a manner such that the neurotrophic agent is transported to the CNS. brain, and/or spinal cord along a neural pathway. A neural pathway includes transport within or along a neuron, through or by way of lymphatics running with a neuron, through or by way of a perivascular space of a blood vessel running with a neuron or neural pathway, through or by way of an adventitia of a blood vessel running with a neuron or neural pathway, or through an hemangiolymphatic system. The invention prefers transport of a neurotrophic agent by way of a neural pathway, rather than through the circulatory system, so that neurotrophic agents that are unable to, or only poorly, cross the blood-brain barrier from the bloodstream into the brain can be delivered to the CNS, brain, and/or spinal cord. The neurotrophic agent, once past the blood-brain barrier and in the CNS, can then be delivered to various areas of the brain or spinal cord through lymphatic channels, through a perivascular space, or transport through or along neurons. In one embodiment, the neurotrophic agent preferably accumulates in areas having the greatest density of receptor or binding sites for that neurotrophic agent.

Use of a neural pathway to transport a neurotrophic agent to the brain, spinal cord or other components of the central nervous system obviates the obstacle presented by the blood-brain barrier so that medications like nerve growth factor (NGF), a protein that cannot normally cross that barrier, can be delivered directly to the brain, cerebellum, brain stem or spinal cord. Although the neurotrophic agent that is administered may be absorbed into the bloodstream as well as the neural pathway, the neurotrophic agent preferably provides minimal effects systemically. In addition, the invention can provide for delivery of a more concentrated level of the neurotrophic agent to neural cells since the neurotrophic agent does not become diluted in fluids present in the bloodstream. As such, the invention provides an improved method of for delivering a neurotrophic agent to the CNS, brain, and/or spinal cord. In addition, delivery of a therapeutic neurotrophic agent to the CNS by a neural pathway can reduce systemic delivery and unwanted systemic side effects. This can maintain whether or not the neurotrophic agent crosses the blood brain barrier.

### The Olfactory Neural Pathway

One embodiment of the present invention includes use in the manufacture of a medicament for use in a manner such that the neurotrophic agent is transported into the CNS, brain, and/or spinal cord along an olfactory neural pathway. Typically, such an embodiment includes administering the neurotrophic agent to tissue innervated by the olfactory nerve and inside the nasal cavity. The olfactory neural pathway innervates primarily the olfactory epithelium in the upper third of the nasal cavity, as described above. Application of the neurotrophic agent to a tissue innervated by the olfactory nerve can deliver the neurotrophic agent to damaged neurons or cells of the CNS, brain, and/or spinal cord. Olfactory neurons innervate this tissue and can provide a direct connection to the CNS, brain, and/or spinal cord due, it is believed, to their role in olfaction.

Delivery through the olfactory neural pathway can employ lymphatics that travel with the olfactory nerve to the pons and other brain areas and from there into dural lymphatics associated with portions of the CNS, such as the spinal cord. Transport along the olfactory nerve can also deliver neurotrophic agents to an olfactory bulb. A perivascular pathway and/or a hemangiolymphatic pathway, such as lymphatic channels running within the adventitia of cerebral blood vessels, can provide an additional mechanism for transport of therapeutic neurotrophic agents to the spinal cord from tissue innervated by the olfactory nerve.

A neurotrophic agent can be administered to the olfactory nerve, for example, through the olfactory epithelium. Such administration can employ extracellular or intracellular (e.g., transneuronal) anterograde and retrograde transport of the neurotrophic agent entering through the olfactory nerves to the brain and its meninges, to the brain stem, or to the spinal cord. Once the neurotrophic agent is dispensed into or onto tissue innervated by the olfactory nerve, the neurotrophic agent may transport through the tissue and travel along olfactory neurons into areas of the CNS including the brain stem, cerebellum, spinal cord, olfactory bulb, and cortical and subcortical structures.

Delivery through the olfactory neural pathway can employ movement of a neurotrophic agent into or across mucosa or epithelium into the olfactory nerve or into a lymphatic, a blood vessel perivascular space, a blood vessel adventitia, or a blood vessel lymphatic that travels with the olfactory nerve to the pons and from there into meningial lymphatics associated with portions of the CNS such as the spinal cord. Blood vessel lymphatics include lymphatic channels that are around the blood vessels on the outside of the blood vessels. This also is referred to as the hemangiolymphatic system. Introduction of a neurotrophic agent into the blood vessel lymphatics does not necessarily introduce the neurotrophic agent into the blood.

### The Trigeminal Neural Pathway

One embodiment of the present invention includes use in the manufacture of a medicament for use a manner such that the neurotrophic agent is transported into the CNS, brain, and/or spinal cord along a trigeminal neural pathway. Typically, such an embodiment includes administering the neurotrophic agent to a portion of the nasal cavity innervated by the trigeminal nerve, as described above. Application of the neurotrophic agent to a tissue innervated by the trigeminal nerve can deliver the neurotrophic agent to damaged neurons or cells of the CNS, brain, and/or spinal cord. Trigeminal neurons innervate the nasal cavity and can provide a direct connection to the CNS, brain, and/or spinal cord due, it is believed, to their role in the common chemical sense including mechanical sensation, thermal sensation and nociception (for example detection of hot spices and of noxious chemicals).

Delivery through the trigeminal neural pathway can employ lymphatics that travel with the trigeminal nerve to the pons and other brain areas and from there into dural lymphatics associated with portions of the CNS, such as the spinal cord. Transport along the trigeminal nerve can also deliver neurotrophic agents to an olfactory bulb. A perivascular pathway and/or a hemangiolymphatic pathway, such as lymphatic channels running within the adventitia of cerebral blood vessels, can provide an additional mechanism for transport of therapeutic neurotrophic agents to the spinal cord from tissue innervated by the trigeminal nerve.

The trigeminal nerve includes large diameter axons, which mediate mechanical sensation, e.g. touch, and small diameter axons, which mediate pain and thermal sensation, both of whose cell bodies are located in the semilunar (or trigeminal) ganglion or the mesencephalic trigeminal nucleus in the midbrain. Certain portions of the trigeminal nerve extend into the nasal cavity. Individual fibers of the trigeminal nerve collect into a large bundle, travel underneath the brain and enter the ventral aspect of the pons. A neurotrophic agent can be administered to the trigeminal nerve, for example, through the nasal cavity's mucosa and/or epithelium. Such administration can employ extracellular or intracellular (e.g., transneuronal) anterograde and retrograde transport of the neurotrophic agent entering through the trigeminal nerves to the brain and its meninges, to the brain stem, or to the spinal cord. Once the neurotrophic agent is dispensed into or onto tissue innervated by the trigeminal nerve, the neurotrophic agent may transport through the tissue and travel along trigeminal neurons into areas of the CNS including the brain stern, cerebellum, spinal cord, olfactory bulb, and cortical and subcortical structures.

Delivery through the trigeminal neural pathway can employ movement of a neurotrophic agent across nasal mucosa or epithelium into the trigeminal nerve or into a lymphatic, a blood vessel perivascular space, a blood vessel adventitia, or a blood vessel lymphatic that travels with the trigeminal nerve to the pons and from there into meningial lymphatics associated with portions of the CNS such as the spinal cord. Blood vessel lymphatics include lymphatic channels that are around the blood vessels on the outside of the blood vessels. This also is referred to as the hemangiolymphatic system. Introduction of a neurotrophic agent into the blood vessel lymphatics does not necessarily introduce the neurotrophic agent into the blood.

### Neural Pathways and Nasal Administration

In one embodiment, the invention can be used to provide delivery by a neural pathway, e.g. a trigeminal or olfactory neural pathway, after administration to the nasal cavity. Upon administration to the nasal cavity, delivery via the trigeminal neural pathway may employ movement of a neurotrophic agent through the nasal mucosa and/or epithelium to reach a trigeminal nerve or a perivascular and/or lymphatic channel that travels with the nerve. Upon administration to the nasal cavity, delivery via the olfactory neural pathway may employ movement of a neurotrophic agent through the nasal mucosa and/or epithelium to reach the olfactory nerve or a perivascular and/or lymphatic channel that travels with the nerve.

For example, the neurotrophic agent can be administered to the nasal cavity in a manner that employs extracellular or intracellular (e.g., transneuronal) anterograde and retrograde transport into and along the trigeminal and/or olfactory nerves to reach the brain, the brain stem, or the spinal cord. Once the neurotrophic agent is dispensed into or onto nasal mucosa and/or epithelium innervated by the trigeminal and/or olfactory nerve, the neurotrophic agent may transport through the nasal mucosa and or epithelium and travel along trigeminal and/or olfactory neurons into areas of the CNS including the brain stem, cerebellum, spinal cord, olfactory bulb, and cortical and subconical structures. Alternatively, administration to the nasal cavity can result in delivery of a neurotrophic agent into a blood vessel perivascular space or a lymphatic that travels with the trigeminal and/or olfactory nerve to the pons, olfactory bulb, and other brain areas, and from there into meningeal lymphatics associated with portions of the CNS such as the spinal cord. Transport along the trigeminal and/or olfactory nerve may also deliver agents administered to the nasal cavity to the olfactory bulb, midbrain, diencephalon, medulla and cerebellum. An agent administered to the nasal cavity can enter the ventral dura of the brain and travel in lymphatic channels within the dura.

In addition, the invention can be carried out in a way that employs a perivascular pathway and/or an hemangiolymphatic pathway, such as a lymphatic channel running within the adventitia of a cerebral blood vessel, to provide an additional mechanism for transport of neurotrophic agent to the spinal cord from the nasal mucosa and/or epithelium. A neurotrophic agent transported by the hemangiolymphatic pathway does not necessarily enter the circulation. Blood vessel lymphatics associated with the circle of Willis as well as blood vessels following the trigeminal and/or olfactory nerve can also be involved in the transport of the neurotrophic agent.

Administration to the nasal cavity employing a neural pathway can deliver a neurotrophic agent to the brain stem, cerebellum, spinal cord and cortical and subcortical structures. The neurotrophic agent alone may facilitate this movement into the CNS, brain, and/or spinal cord. Alternatively, the carrier or other transfer-promoting factors may assist in the transport of the neurotrophic agent into and along the trigeminal and/or olfactory neural pathway. Administration to the nasal cavity of a therapeutic neurotrophic agent can bypass the blood brain barrier through a transport system from the nasal mucosa and/or epithelium to the brain and spinal cord.

### Disorders of the Central Nervous System

The present method can be employed to deliver neurotrophic agents to the brain for diagnosis, treatment or prevention of disorders or diseases of the CNS, brain, and/or spinal cord. These disorders can be neurologic or psychiatric disorders. These disorders or diseases include brain diseases such as Alzheimer's disease, Parkinson's disease, Lewy body dementia, multiple sclerosis, epilepsy, cerebellar ataxia, progressive supranuclear palsy, amyotrophic lateral sclerosis, affective disorders, anxiety disorders, obsessive compulsive disorders, personality disorders, attention deficit disorder, attention deficit hyperactivity disorder, Tourette Syndrome, Tay Sachs, Nieman Pick, and other lipid storage and genetic brain diseases and/or schizophrenia. The invention can also be employed in subjects suffering from or at risk for nerve damage from cerebrovascular disorders such as stroke in the brain or spinal cord, from CNS infections including meningitis and HIV, from tumors of the brain and spinal cord, or from a prion disease. The invention can also be employed to deliver neurotrophic agents to counter CNS disorders resulting from ordinary aging (e.g., anosmia or loss of the general chemical sense), brain injury, or spinal cord injury.

The present invention can be employed to deliver neurotrophic agents to the brain for diagnosis, treatment or prevention of neurodegenerative disorders. Nasal administration of a neurotrophic agent to peripheral nerve cells of the olfactory and/or trigeminal neural pathways innervating the nasal cavity, purported entryway for causative agents of brain diseases, can help protect against disease in these nerve cells and regenerate injured nerve cells, thereby forestalling the subsequent spread of disease to susceptible areas of the CNS, brain, and/or spinal cord.

The application of a neurotrophic agent to the nasal cavity can also help prevent the spread of certain CNS, brain, and/or spinal cord disorders by directly treating peripheral cells and neurons that are injured by neurotoxins and other insults. Prophylactic treatment of these outlying nerve cells helps preclude the entrance of disease-causing agents into the CNS, brain, and/or spinal cord. This method of treatment is particularly beneficial in cases of Alzheimer's disease where an environmental factor is suspected of being one of the causative agents of the disease. Application of a neurotrophic agent to the sensory neurons also in part treats or prevents the loss of smell or of the general chemical sense which may be associated with neurodegenerative diseases and ordinary aging.

The invention may also be used for the prevention of brain disorders particularly in cases where the causative factor enters the brain through olfactory neurons. It is preferred that prophylactic treatments be employed where evidence indicates neuronal degeneration in the olfactory neurons as in the case of Alzheimer's disease and other related brain disorders. Prophylactic treatment of brain disease may involve the direct or indirect application of a neurotrophic agent to the olfactory or nasal epithelium. Such factors may be absorbed into the peripheral olfactory nerve cells to protect those neurons from damage from neurotoxins and other insults and thereby prevent the spread of a disease-causing agent into other areas of the olfactory neural pathway and treat or prevent the loss of smell which may be associated with neurodegenerative diseases and aging.

Treatment of anosmia is another very important potential use for the intranasal olfactory method of delivery. More than 75% of individuals over 80 suffer complete or partial loss of their sense of smell. Many younger individuals including those with Alzheimer's disease and Parkinson's disease also experience losses in olfactory detection. Smell is critical to our experience of taste and thus has significant effects on nutrition.

Treatment of Parkinson's disease may also be an important application of the present invention since the trigeminal nerve pathway can deliver neurotrophic agents from the nasal cavity to the pons in the brain stem. The principal therapeutic target in the brain for Parkinson's is the substantia nigra which extends forward over the dorsal surface of the basis peduncle from the rostral border of the pons toward the subthalamic nucleus. Other therapeutic target areas are the locus ceruleus which is located in the rostral pons region and the ventral tegmental area which is located dorsomedial to the substantia nigra.

An "effective amount" of neurotrophic agent is an amount sufficient to prevent, treat, reduce and/or ameliorate the symptoms and/or underlying causes of any of the above disorders or diseases. In some instances, an "effective amount" is sufficient to eliminate the symptoms of those diseases and, perhaps, overcome the disease itself. In the context of the present invention, the terms "treat" and "therapy" and the like refer to alleviate, slow the progression, prophylaxis, attenuation or cure of existing disease. Prevent, as used herein, refers to putting off, delaying, slowing, inhibiting, or otherwise stopping, reducing or ameliorating the onset of such brain diseases or disorders. It is preferred that a large enough quantity of the neurotrophic agent be applied in non-toxic levels in order to provide an effective level of activity within the neural system against the disease. The method of the present invention may be used with any mammal. Exemplary mammals include, but are not limited to rats, cats, dogs, horses, cows, sheep, pigs, and more preferably humans.

### Articles and Methods of Manufacture

The present invention may be used to provide an article of manufacture providing a neurotrophic agent for administration to the CNS, brain, and/or spinal cord. The article of manufacture can include a vial or other container which contains a composition suitable for the present method together with any carrier, either dried or in liquid form. The article of manufacture further includes instructions in the form of a label on the container and/or in the form of an insert included in a box in which the container is packaged, for the carrying out the method of the invention. The instructions can also be printed on the box in which the vial is packaged. The instructions contain information such as sufficient dosage and administration information so as to allow the subject or a worker in the field to administer the neurotrophic agent. It is anticipated that a worker in the field encompasses any doctor, nurse, technician, spouse, or other care-giver who might administer the neurotrophic agent. The neurotrophic agent can also be self-administered by the subject.

According to the invention, a neurotrophic agent can be used for manufacturing a neurotrophic agent composition or medicament suitable for nasal administration. The invention also relates to methods for manufacturing a neurotrophic agent composition or medicament suitable for nasal administration. For example, a liquid or solid composition can be manufactured in several ways, using conventional techniques. A liquid composition can be manufactured by dissolving a neurotrophic agent in a suitable solvent, such as water, at an appropriate pH, including buffers or other excipients, for example to form a solution described hereinabove.

The present invention may be better understood with reference to the following examples. These examples are intended to be representative of specific embodiments of the invention, and are not intended as limiting the scope of the invention.

### Examples

### Example I - - Intranasal Administration of IGF-I to the Brain

### Introduction

Administering insulin-like growth factor-I (IGF-I) intranasally is an effective means for delivering this neurotrophic agent to the brain of an animal.

### Materials and Methods

Intranasal Delivery to the Brain: Male Sprague-Dawley rats. 200-310 grams, were anesthetized with intraperitoneal pentobarbital (40 mg/kg). Drug delivery to the brain was assessed after intranasal administration of 7.4 nmol of ¹²⁵I-IGF-I in phosphate buffered saline, pH 7.4. Rats were placed on their backs and administered ~25 microliters ¹²⁵-IGF-I to each naris over 10-22 minutes, alternating drops every 2-3 minutes between the left and right nares. Rats subsequently underwent perfusion-fixation within minutes following the completion of ¹²⁵I-IGF-I administration. Perfusion-fixation was performed with 50-100 ml physiologic saline followed by 500 ml of fixative containing 1.25% glutaraldehyde and 1% paraformaldehyde in 0.1 M Sorenson's phosphate buffer, pH 7.4, prior to spinal cord dissection and ¹²⁵I measurement by gamma counting. Areas dissected included the olfactory bulbs, medulla, pons and cerebellum.

### Results

Rapid appearance of radiolabel in the brain was observed with the highest concentrations seen in the olfactory bulbs (3.0±0.47 nM), medulla (0.62±0.16 nM), pons (0.31±0.07 nM and cerebellum (0.30±0.10 nM).

### Conclusions

These results indicate the direct transport of IGF-I along one or more neural pathways into the brain. The high levels of IGF-I in the olfactory bulbs and cerebellum, tissues containing some of the highest amounts of IGF-I mRNA and receptors in the brain, indicate that IGF-I is rapidly transported from the nasal submucosa with preferential accumulation in tissues with the highest receptor levels. Levels of IGF-I as low as 10-100 pM show neuroprotective effects, indicating that the present method delivers effective protective levels of IGF-I to the brain.

### Example 2 - Intranasal Administration of IGF-I to the Spinal Cord

### Introduction

Intranasal administration of protein therapeutics, such as insulin-like growth factor-I (IGF-I) has been shown to be effective in delivering neurotrophic agents to the brain. Intranasal administration circumvents the blood-brain barrier, which otherwise impedes peripherally administered IGF-1. Additionally IGF-I can also be delivered to the spinal cord by intranasal delivery.

### Materials and Methods

Intranasal Delivery to the Spinal Cord: Male Sprague-Dawley rats. 200-310 grams, were anesthetized with intraperitoneal pentobarbital (40 mg/kg). Spinal cord drug delivery was assessed after intranasal administration of 7.4-8.2 nmol of ¹²⁵ I-IGF-I in phosphate buffered saline, pH 7.4. Rats were placed on their backs and administered -25 microliters ¹²⁵I-IGF-I to each naris over 15-20 minutes, alternating drops every 2-3 minutes between left and right nares. Rats subsequently underwent perfusion-fixation within minutes following the completion of ¹²⁵I-IGF-I administration. Perfusion-fixation was performed with 50-100 ml physiologic saline followed by 500 ml of fixative containing 1.25% glutaraldehyde and 1% paraformaldehyde in 0.1 M Sorenson's phosphate buffer, pH 7.4, prior to spinal cord dissection and ¹²⁵I measurement by gamma counting. Areas dissected included the cervical, thoracic and lumbosacral segments of the spinal cord.

Intravenous Delivery to the Spinal Cord: Male Sprague-Dawley rats, 200-310 grams, were anesthetized with intraperitoneal pentobarbital (40 mg/kg). Spinal cord drug delivery was assessed after intravenous administration of 15 pmol of ¹²⁵I-IGF-I in phosphate buffered saline, pH 7.4 in order to assess penetration of ¹²⁵I-IGF-I into the spinal cord from the vascular system. This dose, which resulted in a total blood content of ¹²⁵I-IGF-I comparable to that obtained above with intranasal delivery, was determined by analysis of the area under the blood concentration versus time curve as described by Frey et al. *Drug Delivery* 4:87-92 (1997). Rats were placed on their backs and administered -500 microliters ¹²⁵I-IGF-I over 1 minute via a 24 gauge cannula inserted in the femoral vein. Rats subsequently underwent perfusion-fixation 20 minutes after completion of ¹²⁵I-IGF-I administration. Perfusion-fixation was performed with 50-100 ml physiologic saline followed by 500 ml of fixative containing 1.25% glutaraldehyde and 1% paraformaldehyde in 0.1 M Sorenson's phosphate buffer, pH 7.4. prior to spinal cord dissection and ¹²⁵I measurement by gamma counting. Areas dissected included the cervical, thoracic and lumbosacral segments of the spinal cord.

Intranasal Delivery to the Cerebrospinal Fluid (CSF): Male Sprague-Dawley rats. 200-310 grams, were anesthetized with intraperitoneal pentobarbital (40 mg/kg). Drug delivery to the CSF was determined after intranasal administration of 12.4-12.8 nmol ¹²⁵I-IGF-I in phosphate buffered saline, pH 7.4 in order to assess the mechanism of transport. Rats were placed on their backs and administered -25 microliters ¹²⁵I-IGF-I to each naris over 15-20 minutes, alternating drops every 2-3 minutes between the left and right nares. At two minutes or 17 minutes after completion of the intranasal administration, rats underwent cisternal puncture and 70-100 microliters of CSF were aspirated. ¹²⁵I-IGF-I was then measured in the CSF with gamma counting.

### Results

Rats given ¹²⁵I-IGF-I by intranasal administration showed significant transport of label to spinal cord (see Table 1). Concentrations of ¹²⁵I-IGF-I were highest in the cervical region (approximately 4nM) and exhibited a decreasing gradient caudally with concentration in the thoracic cord of approximately 0.7nM. Table 2 shows a control experiment in which ¹²⁵I-IGF-I was given intravenously with very low concentrations of label reaching the spinal cord (2-3 orders of magnitude lower concentration than that seen following intranasal administration of a dose giving similar blood levels). CSF analysis following intranasal administration failed to show any detectable label in the CSF (see Table 3).

**Table 1**

| Delivery of ¹²⁵I-IGF-I to the Spinal Cord Following Intranasal Administration (Statistical analysis for spinal cord (values listed are ¹²⁵I-IGF-I concentrations)) | | | |
|---|---|---|---|
| ¹²⁵I-IGF-I Administered (nmol) | Cervical Spinal Cord (nM) | Thoracic Spinal Cord (nM) | Blood (nM) |
| 7.4 | 0.46 | 0.09 | 0.27 |
| 7.4 | 3.82 | 1.16 | 0.7 |
| 8.2 | 7.3 | 0.91 | 0.92 |
| mean | 3.86 | 0.72 | 0.63 |
| std error | 1.97 | 0.32 | 0.19 |

**Table 2**

| Intravenous Administration of ¹²⁵I-IGF-I | | | |
|---|---|---|---|
| ¹²⁵I-IGF-I Administered (nmol) | Cervical Spinal Cord (nM) | Thoracic Spinal Cord (nM) | Blood (nM) |
| 14.75 | 0.0062 | 0.0072 | 0.33 |

**Table 3**

| Cerebrospinal Fluid Levels Following Intranasal Administration of ¹²⁵I-IGF | | | |
|---|---|---|---|
| ¹²⁵I-IGF-I Administered (nmol) | CSF Sample (microliters) | CSF (nM) | Blood (nM) |
| 12.8 | 92.5 (17 min. post admin) | ND | 1.6 |
| 12.4 | 67.4 (2 min. post admin) | ND | 1.2 |
| ND - not detected | | | |

### Conclusions

IGF-I is delivered to the spinal cord by intranasal administration. This indicates the usefulness of noninvasive intranasal administration for delivering therapeutic neurotrophic agents to the spinal cord. Intranasal administration of IGF-I did not result in delivery to the cerebrospinal fluid, which indicates a transport system from the nasal submucosa to the brain and spinal cord, exclusive of the cerebrospinal fluid. This suggests that lymphatic channels as well as perhaps hemangiolymphatic pathways, lymphatic channels running within the adventitia of cerebral blood vessels, are the most likely mechanism for transport of IGF-I to the spinal cord from the nasal submucosa.

### Example 3 - - Intranasal Administration of IGF-I via the Trigeminal Nerve

### Introduction

Administering insulin-like growth factor-I (IGF-I) intranasally can deliver this neurotrophic agent to the brain or spinal cord via the trigeminal nerve of an animal.

### Materials and Methods

Male Sprague-Dawley rats, 200-310 grams, were anesthetized with intraperitoneal - pentobarbital (40 mg/kg). Drug delivery to the brain and spinal cord along the trigeminal nerve as assessed after intranasal administration of 7.4-8.2 nmol ¹²⁵-IGF-I in phosphate buffered saline, pH 7.4. Rats were placed on their backs and administered -25 microliters ¹²⁵I-IGF-I to each naris over 15-20 minutes, alternating drops every 2-3 minutes between the left and right nares. Rats subsequently underwent perfusion-fixation within minutes following the completion of ¹²⁵I-IGF-I administration. Perfusion-fixation was performed with 50-100 ml physiologic saline followed by 500 ml of fixative containing 1.25% glutaraldehyde and 1% paraformaldehyde in 0.1 M Sorenson's phosphate buffer, pH 7.4, prior to dissection and ¹²⁵I measurement by gamma counting. Areas dissected included the trigeminal nerve, selected brain areas and spinal cord.

### Results

Radiolabeled IGF was located in the brain, brain stem, spinal cord, and along the trigeminal nerve. Significant amounts of radiolabled IGF-I were observed in the trigeminal nerve of two rats receiving IGF-I intransally. The portion of the nerve closest to the nasal cavity demonstrated the highest concentrations (103-461 nM) with other parts of the nerve closer to the nerve's point of exit from the ventral pons demonstrating significant, but lower concentrations of IGF-I (29-186 nM).

Results from studies conducted according to Examples 1-3 are compiled in Table 4 below. These results show concentrations of IGF-1 in various CNS tissues.

### Conclusions

These results indicate the direct transport of IGF-I along the trigeminal neural pathway.

**Table 4 - -**

| Radiolabel concentration (nM) in different brain regions and blood following intranasal administration of ¹²⁵I-IGF-I. | |
|---|---|
| Brain Region | Concentration (nM) |
| Dorsal Dura | 8 5.9±2 84^{d} |
| Ventral Dura | 91.9±2-23.51^{d} |
| Olfactory Bulb | 5.0±0.96^{a} |
| Anterior Olfactory Nucleus | 2.8±1.0^{b} |
| Frontal Cortex | 1.6±.0.44^{c} |
| Hippocampal Formation | 0.53±0.15^{c} |
| Caudate-Putamen | 0.09±0.09^{c} |
| Dincephalon | 0.54±0.22^{d} |
| Midbrain | 0.51±0.18^{c} |
| Pons | 0.52±0.20^{a} |
| Distal Trigeminal Nerve (V1, V2) | 169.1±61.09^{e} |
| Proximal Trigeminal Nerve (V1, V2) | 151.71±23.0^{e} |
| Trigeminal Nerve (V3) | 99.58±32.76^{e} |
| Trigeminal Nerve Dura | 4136.25^{f} |
| Cerebellum | 0.52±0.22^{a} |
| Medulla | 1.2±0.48^{a} |
| Cervical Spinal Cord | 2.97±1.22^{a} |
| Thoracic Spinal Cord | 0.37±.17^{a} |
| Lumbar Spinal Cord | 0.13±.0.06^{b} |
| Spinal Cord Dura | 9.44±3.11^{b} |

Values are reported as mean = standard error; Trigeminal Dura - mean of two measurements. ^{a}n=7; ^{b}n=6; ^{c}n=5; ^{d}n=4; ^{e}n=3; ^{f}n=2

### Example 4 -- Biological Effects of IGF-I in the CNS After Intranasal Administration

### Introduction

Biological effects of IGF-I were monitored after intranasal administration to demonstrate that this route achieves biologically relevant levels of IGF-I in the CNS

### Materials and Methods

IGF-I was administered generally as described in Examples I-3 above, and at doses of 7.4 nmol and 74 nmol. After the rats were sacrificed, tissues of the CNS were removed, fixed, and analyzed by immunohistochemical methods. The immunohistochemistry was conducted by methods known in the art. The relevant tissue sections were incubated with an antibody recognizing proteins having phosphorylated tyrosine residues followed by staining with DAB. Other CNS tissue was perfused, frozen, and extracted for performing gel electrophoresis and a Western blot, again by methods known in the art.

### Results and Discussion

At both doses, administration of IGF-I increased protein tyrosine phosphorylation in the CNS and increased phosphorylation of IGF-I receptor in the CNS.

IGF-I administration increased protein tyrosine phosphorylation in the olfactory bulb, the cerebellum, and the upper cervical spinal cord. In the olfactory bulb, phosphorylation was clearly evident in the glomerular layer and in mitral cell bodies. In the cerebellum, phosphorylation was clearly evident in the molecular layer and in the purkunje cell layer. In the spinal cord, phosphorylation was clearly evident in the superficial dorsal horn/spinal V^{th} nucleus. IGF-I administration resulted in phosphorylation of the β subunit of the IGF-I receptor and of the protein p^{130cas}.

Observation of these relevant biological effects of IGF-I in the CNS indicates that nasal administration delivers therapeutically effective amounts of this neurotrophic agent to the CNS.

### Example 5 - Intranasal Administration of NGF to the Central Nervous System (Brain and Spinal Cord)

### Introduction

Administering nerve growth factor (NGF) intranasally is an effective means for delivering this neurotrophic agent to the brain and spinal cord of an animal.

### Material and Methods

Male Sprague Dawley rats, 200-310 grams, were anesthetized with intraperitoneal urethane (1.1g/kg), placed in a supine position and then given approximately either 0.4 or 4 nmol of ¹²⁵I-labeled mouse NGF over a period of 30 minutes essentially as described previously by Frey et al. [1997. Drug Delivery 4:87-92]. The ¹²⁵I-NGF was administered as nose drops, 50 µl per naris. Vascular perfusion with physiologic saline and aldehyde fixative was initiated shortly after completion of ¹²⁵I-NGF administration. Selected brain, spinal cord and peripheral tissues were then analyzed for NGF uptake by gamma counting.

### Results

Rapid appearance of radiolabel in the brain and spinal cord was observed. The concentration of ¹²⁵I-NGF was higher in the cervical region of the spinal cord than in the thoracic region and higher in the thoracic region than in the lumbar or sacral regions (Table 4). The concentration found in the spinal cord was dependent on the dose.

| Table 5. Concentration of ¹²⁵I-NGF (pM) In the Spinal Cord Following Olfactory Administration | | | |
|---|---|---|---|
| | R411 | R412 | R416 |
| Dose (nmole) | 0.413 | 3.78 | 4.497 |
| Spinal Cord | | | |
| Cervical | 17 | 700 | 610 |
| Thoracic | 15 | 370 | 350 |
| Lumbar | 11 | 210 | 110 |
| Sacral | 11 | 170 | 120 |

High concentrations of ¹²⁵I-NGF were found in the dura surrounding each of the following: the olfactory bulbs. the dorsal and ventral regions of the brain, the trigeminal nerve and the spinal cord (Tables 5 and 6). The deep cervical lymph nodes contained very high concentrations of ¹²⁵I-NGF as did the common carotid artery.

The trigeminal nerve itself also contained high concentrations of ¹²⁵I-NGF (Table 6).

### Conclusions

The results demonstrate that intranasal administration is an effective method of delivering NGF to the brain, trigeminal nerve and spinal cord. White the earlier studies had clearly demonstrated delivery to the olfactory bulb and other regions of the brain, this is the first demonstration of noninvasive delivery of NGF to the spinal cord. This is important since NGF is unable to cross the blood-brain barrier, and thus has previously not been delivered noninvasively to the spinal cord.

Following intranasal administration, ¹²⁵I-NGF is shown to be in the trigeminal nerve and in the dura that surrounds the trigeminal nerve as well as in the deep cervical lymph nodes. This suggests that intranasally administered NGF moves from the nasal cavity across the nasal mucosa into dural lymphatics that travel along the trigeminal nerve and then into dural lymphatics surrounding the spinal cord. Thus delivery to the spinal cord can occur along the trigeminal neural pathway. The observation of radiolabel in the common carotid and circle of Willis suggests that some transport may also occur through hemangiolymphatic pathways.

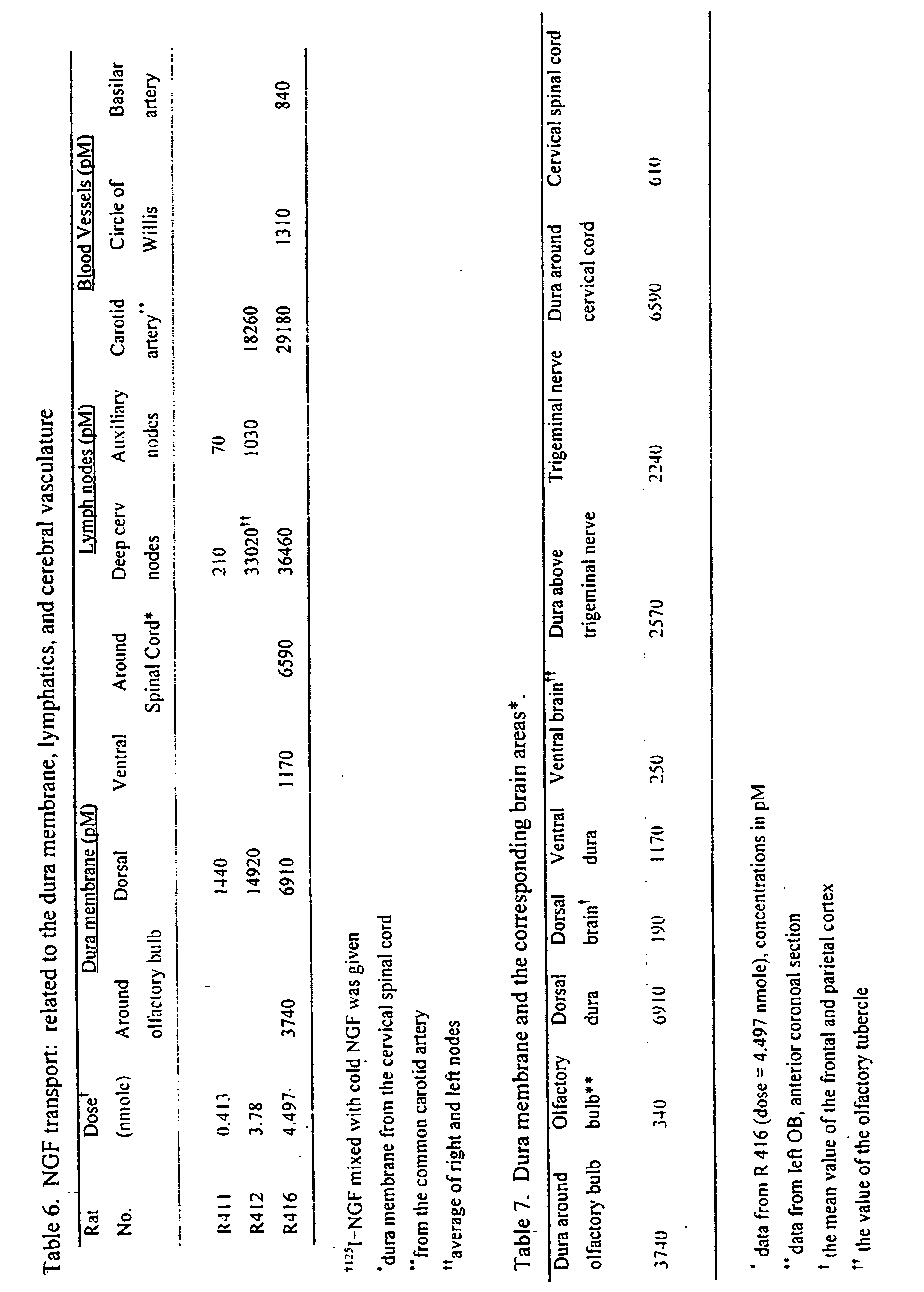

### Example 6 - Dose-Response for Intranasal Administration of NGF to the Brain

### Introduction

Several different doses of NGF were administered intranasally to determine the effect of the dose on the levels of NGF achieved in the brain.

### Materials and Methods

These experiments were conducted generally as described in Example 4. The dose of labeled NGF ranged from 2.4 to 38 nmol.

### Results

The levels of NGF in several brain tissues at doses between 2.4 to 38 nmol are illustrated in Figures 1 - 5.

### Discussion

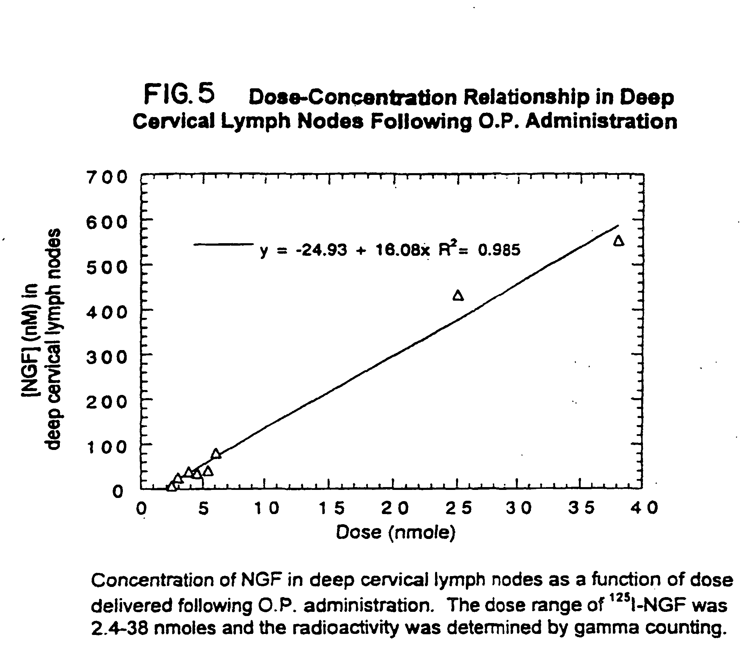

A linear relationship was found between NGF concentrations and the doses given by nasal administration in the following brain regions: olfactory bulb and its dura membrane (Figure 1 and Figure 2), olfactory epithelium (Figure 3), cervical spinal cord (Figure 4) and deep cervical lymph nodes (Figure 5), within the dose range of 2.4 - 38 nmoles. In addition, the concentration of NGF in the olfactory bulb was found to correlate well with the levels of NGF in both the olfactory epithelium and the olfactory bulb dura. The linear dose-concentration relationship observed in this study does not necessarily mean that the receptor-mediated, saturable process is not involved in NGF-transport. The dose of NGF tested may not be high enough to saturate the NGF receptors in the brain.

### Example 7 - Intranasal Administration of FGF to the Central Nervous System (Brain and Spinal Cord)

### Introduction

Administering fibroblast growth factor (FGF) intranasally is an effective means for delivering this neurotrophic agent to the brain and spinal cord of an animal.

### Materials and Methods

Basic FGF (bFGF) was obtained by methods described hereinabove. The ¹²⁵I-bFGF was intranasally administered to rats generally as described in the previous examples for IGF and NGF. After sacrifice, the rat brain was processed for determining the quantity of FGF in different sections of the brain, both by counting labeled FGF, and by phosphor scanning employing a Packard Cyclone Phosphor Scanner. The scanning was conducted on a saggital section through the lateral 1 mm of the brain. In addition, certain portions of the CNS were separated and the concentration of FGF was determined in these separate portions.

### Results

The scanning indicated that substantial levels of bFGF were delivered to the olfactory bulb, cerebellum, and frontal cortex. Scanning also indicated little bFGF in the olfactory tract and the ventral area of the brain.

The concentrations of FGF observed in separate portions of the CNS were: 1200 pM in the olfactory bulb, 1600 pM in the upper cervical spinal cord. In addition, about 200 to about 700 pM bFGF were observed in other regions of the brain. Concentrations of bFGF of about 2,000 to about 6000 pM were observed in the trigeminal nerve.

### Conclusions

The results demonstrate that intranasal administration is an effective method of delivering FGF to the brain, trigeminal nerve and spinal cord. The concentrations delivered are in excess of those believed necessary for biological activity of FGF (10-100 pM). The high concentration of FGF in the trigeminal nerve suggests that this agent is delivered by transport along a trigeminal nerve pathway.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

All publications and patent applications in this specification are indicative of the level of ordinary skill in the art to which this invention pertains. The invention has been described with reference to various specific and preferred embodiments and techniques.

## Claims

1. Use of a neurotrophic agent or biologically active variant thereof in the manufacture of a medicament for delivering a therapeutically effective amount of said neurotrophic agent or said variant thereof to the central nervous system of a mammal by way of a nasal cavity, said medicament comprising a unit dose of 0.1 nmol to 1000 nmol of said neurotrophic agent or said variant thereof, wherein administration of said medicament to said nasal cavity of said mammal provides for transport of said neurotrophic agent or said variant thereof to the central nervous system of said mammal in an amount effective to provide a protective or therapeutic effect on a cell of the central nervous system, said neurotrophic agent being selected from the group consisting of an insulin-like growth factor (IGF), a nerve growth factor (NGF), and a fibroblast growth factor (FGF), and said biologically active variant thereof having at least 70% amino acid sequence identity with the amino acid sequence for said neurotrophic agent, and wherein said biologically active variant of IGF is a biologically active variant of insulin-like growth factor-1 (IGF-1).

2. Use according to claim 1, wherein administration of said medicament to said nasal cavity results in a therapeutically effective amount of 10⁻¹¹ M to about 10⁻⁹ M of said neurotrophic agent or said variant thereof in a portion of the central nervous system.

3. Use according to claim 1 or 2, wherein administration of said medicament to said nasal cavity provides for transport of said neurotrophic agent or said variant thereof to lymphatics associated with the central nervous system.

4. Use according to claim 1 or 2, wherein administration of said medicament to said nasal cavity provides for transport of said neurotrophic agent or said variant thereof to an olfactory bulb, a hippocampal formation, a frontal cortex, a midbrain, a brainstem, a spinal cord, or a combination thereof.

5. Use according to any one of the preceding claims, wherein the medicament comprises a liquid, a powder, a spray, a gel, an ointment, an infusion, or a combination thereof.

6. Use according to any one of the preceding claims, wherein the medicament comprises a substance having an affinity for a receptor site on a neuron.

7. Use according to any one of the preceding claims, wherein the medicament comprises a pharmaceutically-acceptable carrier, a lipophilic micelle, a liposome, or a combination thereof.

8. Use according to claim 7, wherein the lipophilic micelle or liposome comprises a ganglioside, a phosphatidylcholine, a phosphatidylserine, lipofectin, DOTAP, or a combination thereof.

9. Use according to any one of the preceding claims, wherein the medicament comprises a controlled release polymer.

10. Use according to claim 9, wherein the polymer comprises poly(ethylene-co-vinyl acetate).

11. Use according to any one of the preceding claims, wherein said administration comprises applying the medicament to an upper third of the nasal cavity.

12. Use according to claim 11, wherein said administration comprises applying the medicament to an olfactory area in the upper third of the nasal cavity.

13. Use according to claim 11, wherein said administration comprises applying the medicament to a roof of a nose.

14. Use according to any one of the preceding claims, wherein said mammal is a human.

15. Use according to claim 14, wherein said medicament comprises about 1 nmol to about 1000 nmol of said neurotrophic agent or said variant thereof.

16. Use according to claim 15, wherein said medicament comprises about 1 nmol to about 300 nmol of said neurotrophic agent or said variant thereof.

17. Use according to claim 15, wherein said medicament comprises about 300 nmol to about 700 nmol of said neurotrophic agent or said variant thereof.

18. Use according to claim 15, wherein said medicament comprises about 700 nmol to about 1000 nmol of said neurotrophic agent or said variant thereof.

19. Use according to any one of the preceding claims, wherein said insulin-like growth factor is insulin-like growth factor-I (IGF-I).

20. Use according to claim 19, wherein said IGF-I is human IGF-I, and said biologically active variant thereof has at least 70% amino acid sequence identity with the amino acid sequence for human IGF-I.

21. Use according to any one of claims 1 to 18, wherein said FGF is basic FGF (FGF-2).

22. Use according to claim 21, wherein said FGF-2 is human FGF-2, and said biologically active variant thereof has at least 70% amino acid sequence identity with the amino acid sequence for human FGF-2.

23. Use according to any one of claims 1 to 18, wherein said NGF is human NGF, and said biologically active variant thereof has at least 70% amino acid sequence identity with the amino acid sequence for human NGF.

24. Use according to any one of claims claim 19-23, wherein said neurotrophic agent or said variant thereof is recombinantly produced.

25. Use according to any one of the preceding claims, wherein administration of said medicament to said nasal cavity provides for transport of said neurotrophic agent or said variant thereof to the central nervous system of the mammal in an amount effective for treating a neurological condition, a central nervous system disorder, a psychiatric disorder, or a combination thereof.

26. Use according to claim 25, wherein the condition or disorder comprises a neurodegenerative disorder, an affective disorder, nerve damage from a cerebrovascular disorder, a CNS infection, or a combination thereof.

27. Use according to claim 25, wherein the condition or disorder comprises Alzheimer's disease, Parkinson's disease, Lewy body dementia, multiple sclerosis, cerebellar ataxia, progressive supranuclear palsy, amyotrophic lateral sclerosis, affective disorders, anxiety disorders, schizophrenia, stroke in the brain, stroke in the spinal cord, meningitis, HIV infection of the central nervous system, a tumor of the brain, a tumor of the spinal cord, a prion disease, anosmia, brain injury, spinal cord injury, or a combination thereof.

## Patentansprüche

1. Verwendung eines neurotropischen Wirkstoffs oder einer biologisch aktiven Variante davon bei der Herstellung eines Medikaments zur Abgabe einer therapeutisch wirksamen Menge des neurotropischen Wirkstoffs oder der Variante davon über die Nasenhöhle an das Zentralnervensystem eines Säugers, wobei das Medikament einer Einzeldosis von 0,1 nmol bis 1000 nmol des neurotropischen Wirkstoffs oder der Variante davon umfasst, wobei eine Verabreichung des Medikaments an die Nasenhöhle des Säugers für einen Transport des neurotropischen Wirkstoffs oder der Variante davon an das Zentralnervensystem in einer Menge sorgt, die eine schützende oder therapeutische Wirkung auf eine Zelle des Zentralnervensystems liefert, wobei der neurotropische Wirkstoff aus der Gruppe, bestehend aus insulinartigem Wachstumsfaktor (IGF), Nervenwachstumsfaktor (NGF) und Fibroblastenwachstumsfaktor (FGF), ausgewählt ist und die biologisch aktive Variante davon wenigstens 70% Aminosäuresequenz-Identität mit der Aminosäuresequenz für den neurotropischen Wirkstoff hat und wobei die biologisch aktive Variante von IGF eine biologisch aktive Variante von insulinartigem Wachstumsfaktor I (IGF-I) ist.

2. Verwendung nach Anspruch 1, wobei eine Verabreichung des Medikaments an die Nasenhöhle in einer therapeutisch wirksamen Menge von 10⁻¹¹ M bis etwa 10⁻⁹ M des neurotropischen Wirkstoffs oder der Variante davon in einem Teil des Zentralnervensystems resultiert.

3. Verwendung nach Anspruch 1 oder 2, wobei eine Verabreichung des Medikaments an die Nasenhöhle für einen Transport des neurotropischen Wirkstoffs oder der Variante davon an mit dem Zentralnervensystem verbundene Lymphgefäße sorgt.

4. Verwendung nach Anspruch 1 oder 2, wobei eine Verabreichung des Medikaments an die Nasenhöhle für einen Transport des neurotropischen Wirkstoffs oder der Variante davon zum Bulbus olfactorius, zur Formatio hippocampi, zum frontalen Cortex, zum Mittelhirn, zum Stammhirn, zum Rückenmark oder einer Kombination davon sorgt.

5. Verwendung nach einem der vorangehenden Ansprüche, wobei das Medikament eine Flüssigkeit, ein Pulver, ein Spray, ein Gel, eine Salbe, eine Infusion oder eine Kombination davon umfasst.

6. Verwendung nach einem der vorangehenden Ansprüche, wobei das Medikament eine Substanz umfasst, die Affinität für eine Rezeptorstelle an einem Neuron hat.

7. Verwendung nach einem der vorangehenden Ansprüche, wobei das Medikament einen pharmazeutisch annehmbaren Träger, eine lipophile Mizelle, ein Liposom oder eine Kombination davon umfasst.

8. Verwendung nach Anspruch 7, wobei die lipophile Mizelle oder das Liposom ein Gangliosid, ein Phosphatidylcholin, ein Phosphatidylserin, Lipofectin, DOTAP oder eine Kombination davon umfasst.

9. Verwendung nach einem der vorangehenden Ansprüche, wobei das Medikament ein Polymer zur kontrollierten Freisetzung umfasst.

10. Verwendung nach Anspruch 9, wobei das Polymer Poly(ethylen-co-vinylacetat) umfasst.

11. Verwendung nach einem der vorangehenden Ansprüche, wobei die Verabreichung Anwenden des Medikaments auf ein oberes Drittel der Nasenhöhle umfasst.

12. Verwendung nach Anspruch 11, wobei die Verabreichung Anwenden des Medikaments auf einen Bereich des Geruchssinns im oberen Drittel der Nasenhöhle umfasst.

13. Verwendung nach Anspruch 11, wobei die Verabreichung Anwenden des Medikaments auf ein Nasendach umfasst.

14. Verwendung nach einem der vorangehenden Ansprüche, wobei der Säuger ein Mensch ist.

15. Verwendung nach Anspruch 14, wobei das Medikament etwa 1 nmol bis etwa 1000 nmol des neurotropischen Wirkstoffs oder der Variante davon umfasst.

16. Verwendung nach Anspruch 15, wobei das Medikament etwa 1 nmol bis etwa 300 nmol des neurotropischen Wirkstoffs oder der Variante davon umfasst.

17. Verwendung nach Anspruch 15, wobei das Medikament etwa 300 nmol bis etwa 700 nmol des neurotropischen Wirkstoffs oder der Variante davon umfasst.

18. Verwendung nach Anspruch 15, wobei das Medikament etwa 700 nmol bis etwa 1000 nmol des neurotropischen Wirkstoffs oder der Variante davon umfasst.

19. Verwendung nach einem der vorangehenden Ansprüche, wobei der insulinartige Wachstumsfaktor insulinartiger Wachstumsfaktor-I (IGF-I) ist.

20. Verwendung nach Anspruch 19, wobei der IGF-I humaner IGF-I ist, und die biologisch aktive Variante davon wenigstens 70% Aminosäuresequenz-Identität mit der Aminosäuresequenz für humanen IGF-I hat.

21. Verwendung nach einem der Ansprüche 1 bis 18, wobei der FGF basischer FGF (FGF-2) ist.

22. Verwendung nach Anspruch 21, wobei der FGF-2 humaner FGF-2 ist, und die biologisch aktive Variante davon wenigstens 70% Aminosäuresequenz-Identität mit der Aminosäuresequenz für humanen FGF-2 hat.

23. Verwendung nach einem der Ansprüche 1 bis 18, wobei der NGF humaner NGF ist, und die biologisch aktive Variante davon wenigstens 70% Aminosäuresequenz-Identität mit der Aminosäuresequenz für humanen NGF hat.

24. Verwendung nach einem der Ansprüche 19 bis 23, wobei der neurotropische Wirkstoff oder die Variante davon rekombinant produziert ist.

25. Verwendung nach einem der vorangehenden Ansprüche, wobei eine Verabreichung des Medikaments an die Nasenhöhle für einen Transport des neurotropischen Wirkstoffs oder der Variante davon an das Zentralnervensystem des Säugers in einer Menge sorgt, die für eine Behandlung eines neurologischen Krankheitsbilds, einer Störung des Zentralnervensystems, einer psychiatrischen Störung oder einer Kombination davon wirksam ist.

26. Verwendung nach Anspruch 25, wobei das Krankheitsbild oder die Störung eine neurodegenerative Störung, eine Affektstörung, eine Nervenschädigung durch eine cerebrovaskuläre Störung, eine ZNS-Infektion oder eine Kombination davon umfasst.

27. Verwendung nach Anspruch 25, wobei das Krankheitsbild oder die Störung Alzheimer'sche Krankheit, Parkinson-Krankheit, Lewy-Körper-Demenz, multiple Sklerose, cerebrale Ataxie, progressive supranukleäre Lähmung, amyotrophe Lateralsklerose, Affektstörungen, Angststörungen, Schizophrenie, Hirnschlag, Schlaganfall im Rückenmark, Meningitis, HIV-Infektion des Zentralnervensystems, einen Tumor im Gehirn, einen Tumor im Rückenmark, eine Prionenkrankheit, Anosmie, Hirnverletzung, Rückenmarksverletzung oder eine Kombination davon umfasst.

## Revendications

1. Utilisation d'un agent neurotrophique ou d'une variante biologiquement active de celui-ci dans la fabrication d'un médicament pour délivrer une quantité thérapeutiquement efficace dudit agent neurotrophique ou de ladite variante de celui-ci au système nerveux central d'un mammifère au moyen d'une cavité nasale, ledit médicament comprenant une dose unitaire de 0,1 nmole à 1000 nmoles dudit agent neurotrophique ou de ladite variante de celui-ci, dans laquelle l'administration dudit médicament à ladite cavité nasale dudit mammifère permet de transporter ledit agent neurotrophique ou ladite variante de celui-ci au système nerveux central dudit mammifère en une quantité efficace pour fournir un effet protecteur ou thérapeutique à une cellule du système nerveux central, ledit agent neurotrophique étant choisi dans le groupe constitué par un facteur de croissance semblable à l'insuline (IGF), un facteur de croissance neuronal (NGF) et un facteur de croissance fibroblastique (FGF), et ladite variante biologiquement active de celui-ci ayant une identité de séquence des acides aminés d'au moins 70 % avec la séquence des acides aminés dudit agent neurotrophique, et dans laquelle ladite variante biologiquement active d'IGF est une variante biologiquement active du facteur de croissance semblable à l'insuline-I (IGF-1).

2. Utilisation selon la revendication 1, dans laquelle l'administration dudit médicament à ladite cavité nasale conduit à une quantité thérapeutiquement efficace de 10⁻¹¹ M à environ 10⁻⁹ M dudit agent neurotrophique ou de ladite variante de celui-ci dans une partie du système nerveux central.

3. Utilisation selon la revendication 1 ou 2, dans laquelle l'administration dudit médicament à ladite cavité nasale permet le transport dudit agent neurotrophique ou de ladite variante de celui-ci au système lymphatique associé avec le système nerveux central.

4. Utilisation selon la revendication 1 ou 2, dans laquelle l'administration dudit médicament à ladite cavité nasale permet le transport dudit agent neurotrophique ou de ladite variante de celui-ci à un bulbe olfactif, une formation hippocampique, un cortex frontal, un cerveau intermédiaire, un tronc cérébral, une moelle épinière ou une combinaison de ceux-ci.

5. Utilisation selon l'une quelconque des revendications précédentes, dans laquelle le médicament comprend un liquide, une poudre, une pulvérisation, un gel, une pommade, une infusion ou une combinaison de ceux-ci.

6. Utilisation selon l'une quelconque des revendications précédentes, dans laquelle le médicament comprend une substance ayant une affinité pour un site récepteur sur un neurone.

7. Utilisation selon l'une quelconque des revendications précédentes, dans laquelle le médicament comprend un véhicule pharmaceutiquement acceptable, une micelle lipophile, un liposome ou une combinaison de ceux-ci.

8. Utilisation selon la revendication 7, dans laquelle la micelle lipophile ou le liposome comprend un ganglioside, une phosphatidylcholine, une phosphatidyl-sérine, de la lipofectine, DOTAP ou une combinaison de ceux-ci.

9. Utilisation selon l'une quelconque des revendications précédentes, dans laquelle le médicament comprend un polymère à libération régulée.

10. Utilisation selon la revendication 9, dans laquelle le polymère comprend du poly(éthylène-co-acétate de vinyle).

11. Utilisation selon l'une quelconque des revendications précédentes, dans laquelle ladite administration comprend l'application du médicament au tiers supérieur de la cavité nasale.

12. Utilisation selon la revendication 11, dans laquelle ladite administration comprend l'application du médicament à une zone olfactive du tiers supérieur de la cavité nasale.

13. Utilisation selon la revendication 11, dans laquelle ladite administration comprend l'application du médicament à la voûte du nez.

14. Utilisation selon l'une quelconque des revendications précédentes, dans laquelle ledit mammifère est un humain.

15. Utilisation selon la revendication 14, dans laquelle ledit médicament comprend environ 1 nmole à environ 1000 nmoles dudit agent neurotrophique ou de ladite variante de celui-ci.

16. Utilisation selon la revendication 15, dans laquelle ledit médicament comprend environ 1 nmole à environ 300 nmoles dudit agent neurotrophique ou de ladite variante de celui-ci.

17. Utilisation selon la revendication 15, dans laquelle ledit médicament comprend environ 300 nmoles à environ 700 nmoles dudit agent neurotrophique ou de ladite variante de celui-ci.

18. Utilisation selon la revendication 15, dans laquelle ledit médicament comprend environ 700 nmoles à environ 1000 nmoles dudit agent neurotrophique ou de ladite variante de celui-ci.

19. Utilisation selon l'une quelconque des revendications précédentes, dans laquelle ledit facteur de croissance semblable à l'insuline est le facteur de croissance semblable à l'insuline-I (IGF-1).

20. Utilisation selon la revendication 19, dans laquelle ledit IGF-I est un IGF-I humain, et ladite variante biologiquement active de celui-ci a une identité de séquence des acides aminés d'au moins 70 % avec la séquence des acides aminés de l'IGF-I humain.

21. Utilisation selon l'une quelconque des revendications 1 à 18, dans laquelle ledit FGF est FGF basique (FGF-2).

22. Utilisation selon la revendication 21, dans laquelle ledit FGF-2 est FGF-2 humain, et ladite variante biologiquement active de celui-ci a une identité de la séquence des acides aminés d'au moins 70 % avec la séquence des acides aminés de FGF-2 humain.

23. Utilisation selon l'une quelconque des revendications 1 à 18, dans laquelle ledit NGF est NGF humain, et ladite variante biologiquement active de celui-ci a une identité de séquence des acides aminés d'au moins 70 % avec la séquence des acides aminés de NGF humain.

24. Utilisation selon l'une quelconque des revendications 19 à 23, dans laquelle ledit agent neurotrophique ou ladite variante de celui-ci est produit de façon recombinante.

25. Utilisation selon l'une quelconque des revendications précédentes, dans laquelle l'administration dudit médicament à ladite cavité nasale permet le transport dudit agent neurotrophique ou de ladite variante de celui-ci au système nerveux central du mammifère en une quantité efficace pour traiter une affection neurologique, un trouble du système nerveux central, un trouble psychiatrique, ou une combinaison de ceux-ci.

26. Utilisation selon la revendication 25, dans laquelle l'affection ou le trouble comprend un trouble neurodégénératif, un trouble affectif, une lésion nerveuse provenant d'un trouble cérébrovasculaire, une infection du SNC, ou une combinaison de ceux-ci.

27. Utilisation selon la revendication 25, dans laquelle l'affection ou le trouble comprend une maladie d'Alzheimer, une maladie de Parkinson, une démence à corps de Lewy, une sclérose en plaques, une ataxie cérébelleuse, une paralysie supranucléaire progressive, une sclérose latérale amyotrophique, des troubles affectifs, des troubles anxieux, une schizophrénie, une attaque cérébrale, une attaque de la moelle épinière, une méningite, une infection du système nerveux central par VIH, une tumeur cérébrale, une tumeur de la moelle épinière, une maladie à prion, une anosmie, une lésion cérébrale, une lésion de la moelle épinière, ou une combinaison de ceux-ci.
